# EUROPÄISCHE PATENTSCHRIFT

(11) **EP 1 091 696 B1**
(45) Veröffentlichungstag und Bekanntmachung des Hinweises auf die Patenterteilung: **08.09.2004**
(21) Anmeldenummer: 98928043.3
(22) Anmeldetag: 29.06.1998
(51) Int. Cl.: A61B 17/15

(54) **VORRICHTUNG ZUM EINSETZEN EINER KNIEPROTHESE**
DEVICE FOR INSERTING A PROSTHETIC KNEE
DISPOSITIF POUR INSERER UNE PROTHESE DU GENOU

(43) Veröffentlichungstag der Anmeldung: 18.04.2001
(73) Patentinhaber: PLUS ENDOPROTHETIK AG, 6343 Rotkreuz (CH)
(72) Erfinder: HAURI, Bernhard, CH-5053 Staffelbach (CH); HAURI, Thomas, CH-5053 Staffelbach (CH); BERNER, Werner, CH-5018 Erlinsbach (CH)
(74) Vertreter: Patentanwälte Schaad, Balass, Menzl & Partner AG
(86) Internationale Anmeldenummer: PCT/CH1998/000280
(87) Internationale Veröffentlichungsnummer: WO 2000/000093

(56) Entgegenhaltungen:
- EP-A- 0 322 363
- EP-A- 0 691 110
- EP-A- 0 709 061
- WO-A-94/08528
- FR-A- 2 679 766
- US-A- 4 457 307
- US-A- 4 574 794
- US-A- 5 514 143

## Beschreibung

Die vorliegende Erfindung betrifft eine Vorrichtung, welche es einem Chirurgen erlaubt, bei der Implantation einer Kniegelenktotalendoprothese die Resektion des Femurs und der Tibia in äusserst präziser Weise durchzuführen.

Die exakte Lage der Resektionslinien an Femur und Tibia sind für eine lange Lebensdauer einer Kniegelenktotalendoprothese von entscheidender Bedeutung. Bislang ist die Durchführung der Resektion selbst für einen erfahrenen Chirurgen äusserst anspruchsvoll, gilt es doch durch die Operation die Normanlageflächen entsprechend der Vorgabe der Geometrie der Endoprothese zu schaffen, dabei die Normanlageflächen entsprechend der gewünschten mechanischen Beinachsen auszurichten wobei gegebenenfalls auch pathologische Fehlstellungen zu korrigieren sind, und zudem die Lage und die Wirkung der vorhandenen Bänder und Muskeln zu berücksichtigen. Die Ausrichtung von Tibia und Femur erfolgt üblicherweise durch Inaugenscheinnahme unter allfälliger Zuhilfenahme von intra- oder extramedullären Hilfsmitteln, wobei als zusätzliche Behinderung das Operationsfeld oft einen erschwerten Zugang aufweist. Diese Randbedingungen können selbst bei Chirurgen mit grosser Erfahrung Stressituationen verursachen.

Eine Kniegelenktotalendoprothese besteht aus einer am Femur und einer an der Tibia befestigten Komponente. Bevor die Eniegelenktotalendoprothese implantiert werden kann müssen die angrenzenden Knochenbereiche des Femurs und der Tibia in geeigneter Weise reseziert werden, um Normanlageflächen entsprechend der Geometrie der Endoprothesen zu schaffen. Üblicherweise werden die Frontalen der Tibia und des Femurs reseziert. Wenigstens der Femur erhält darüber hinaus zumindest einen sogenannten Dorsal- sowie einen Ventralschnitt, da der Femurteil von Totalendoprothesen üblicherweise u-förmig ausgestaltet ist. Die im allgemeinen von Herstellern von Knieprothesen angebotenen Instrumente erlauben nicht die erforderlichen Knochenschnitte am Femur und an der Tibia mit der erforderlichen Genauigkeit vorzunehmen.

Als weitere wesentliche Forderung gilt es jedoch zu berücksichtigen, dass die bei der Biegung und Streckung des Knies aneinander gleitenden Komponenten der Knieprothese immer die richtige Stellung zueinander haben, d.h. dass die mechanische Beinachse maximal 3° Varus oder 3° Valgus von der physiologischen Beinachse abweichen darf, bevorzugt beträgt die Abweichung weniger als ± 2°. Zudem gilt es zu berücksichtigen, dass die flexible Verbindung zwischen den beiden Komponenten durch Bänder und Muskeln bewirkt wird, soweit diese bei der Implantation der Prothese erhalten bleiben. Dies erfordert eine Equilibrierung des Bandapparates, welche sowohl bei der Extension als auch bei der Flexion eine gute Stabilität des Kniegelenks bewirkt.

Die bekannten Instrumentarien für die Implantation von Kniegelenktotalendoprothesen umfassen in der Regel folgende Kittel:
- Mittel zur Ausrichtung der Tibia bezüglich dem Femur zur Erzielung der gewünschten Beinachsenstellung;
- Mittel zur Herstellung der gewünschten Spannung der Kniebänder;
- Mittel zur Durchführung der Resektion von Tibia und Femur, in Form von Schneidlehren, welche der Führung eines Sägeblattes dienen.

Ein derartiges Instrumentarium ist aus der Druckschrift EP 0 322 363 A1 bekannt. Dieses Instrumentarium verwendet ein extramedulläres Mittel zum Ausrichten von Tibia und Femur (extramedullary alignment system) und weist den Nachteil auf, dass die Ausrichtung des Femurs nur mit Hilfe eines Röntgenapparates bestimmbar ist. Zudem erfolgt die Befestigung des Bezugssystems für die Knochenschnitte nach Augenmass, wobei das Bezugssystem zudem den Zugang zum Operationsfeld erschwert.

Ein weiteres Instrumentarium ist aus der Druckschrift EP 0 691 110 A2 bekannt. Dieses Instrumentarium verwendet ein intramedulläres Mittel zum Ausrichten von Tibia und Femur (intramedullary alignment system) und weist den Nachteil auf, dass zur gegenseitigen Fixation von Tibia und Femur je ein Führungsspiess erforderlich ist, welcher in den Tibia- bzw. Femurmarkraum eingeführt wird. Dieser Eingriff in den Markraum kann Thrombosen bzw. Embolien verursachen, was sich letal auswirken kann.

Eine Vorrichtung gemäß der Präambel von Anspruch 1 ist aus der US-A-4 457 307 bekannt.

Es ist Aufgabe der vorliegenden Erfindung eine Vorrichtung zur Festlegung von Resektionsschnitten am Femur sowie an der Tibia zur Vorbereitung einer Implantation einer Kniegelenktotalendoprothese zu schaffen, welches einfach und zuverlässig reproduzierbar durchzuführen ist.

Diese Aufgabe wird gelöst mit einer Vorrichtung aufweisend die Merkmale von Anspruch 1. Die Unteransprüche 2 bis 21 beziehen sich auf weitere vorteilhafte Ausgestaltungen der erfindungsgemässen Vorrichtung.

Die erfindungsgemässe Vorrichtung umfasst in einer vorteilhaften Ausgestaltung eine Referenzvorrichtung bestehend im wesentlichen aus einem lösbar im distalen Bereich des Femurs arretierbaren Basisteil sowie einem gelenkig und/oder verschiebbar mit dem Basisteil verbundenen Referenzkörper, welcher ein Koordinatensystem X,Y,Z bestimmende Mittel aufweist, wobei die Ausrichtung des Referenzkörpers bezüglich dem Femur lagegenau positionierbar ist und wobei ein zwischen dem Referenzkörper und dem Basisteil wirkendes Betätigungsmittel zum Fixieren deren gegenseitiger Lage vorgesehen ist, und wobei die das Koordinatensystem X,Y,Z bestimmende Mittel zum ausgerichteten Befestigen von Bearbeitungsmitteln wie einer Schneidlehre, eines Basisbalkens oder einer Messvorrichtung ausgestaltet sind.

Ein Vorteil dieser Vorrichtung ist darin zu sehen, dass die Referenzvorrichtung fest mit dem Femur verbunden und vorzugsweise in Verlaufsrichtung der Belastungsachse des Femurs ausgerichtet ist, und dass alle Schnitte an Femur und Tibia bezüglich diesem Referenzsystem ausgerichtet getätigt werden, so dass sehr präzis und definiert ausgerichtet verlaufende Resektionsschnitte bzw. Resektionsflächen an Femur und Tibia erstellbar sind.

Die Referenzvorrichtung ist mit einer Vielzahl von unterschiedlich ausgestalteten Mitteln am Femur befestigbar, so beispielsweise mit Knochenschrauben, oder den Femur zumindest teilweise umfassende Greifarme, welche zur besseren Verankerung zudem Dornen aufweisen können, welche in den Femur eindringen.

Die erfindungsgemässe Vorrichtung umfasst in einer weiteren vorteilhaften Ausgestaltung eine extramedullär und lösbar im distalen Bereich des Femurs befestigbare Referenzvorrichtung, deren Ausrichtung bezüglich dem Femur lagegenau positionierbar ist, sowie eine extramedullär und lösbar an der Tibia befestigbare Tibiaschiene, wobei die Ausrichtung der Tibiaschiene bezüglich der Tibia lagegenau positionierbar ist, sowie eine die Referenzvorrichtung sowie die Tibiaschiene lösbar fest verbindende Befestigungsvorrichtung.

Diese erfindungsgemässe Ausführungsform weist den Vorteil auf, dass die Tibia bezüglich dem Femur in eine genau definierte Lage gebracht und danach fixiert werden kann. Daher kann die Verlaufsrichtung der Resektionsschnitte an der Tibia über die am Femur befestigte Schneidvorrichtung vorgegeben werden. Die Stellung der Tibia kann bezüglich dem Femur genau eingestellt werden, um beispielsweise den Verlauf der mechanischen Beinachse zu korrigieren. In einer vorteilhaften Ausgestaltung ist die Befestigungsvorrichtung U-förmig oder rechteckförmig ausgestaltet, so dass der Operationsbereich am Knie auch bei angebrachter Befestigungsvorrichtung weitgehend frei zugänglich ist.

Die erfindungsgemässe Vorrichtung umfasst in einer weiteren vorteilhaften Ausgestaltung eine lösbar am distalen Bereich des Femurs arretierbare Referenzvorrichtung, deren Ausrichtung bezüglich dem Femur lagegenau positionierbar ist, sowie eine mit der Referenzvorrichtung beweglich verbundene Schneidvorrichtung, insbesondere eine Schneidlehre zum Führen eines Sägeblattes oder eine Sägevorrichtung mit einem Sägeblatt, wobei die Ausrichtung der Schneidvorrichtung, insbesondere des Sägeblattes zumindest durch die Ausrichtung der Referenzvorrichtung bestimmt ist. Es lassen sich Schneidvorrichtungen mit unterschiedlichsten Schneidverfahren verwenden, zum Beispiel Sägen, Ultraschalltrennvorrichtungen oder auch die Verwendung von Laser. Die erfindungsgemässe Vorrichtung erlaubt die Schneidvorrichtung derart zu führen, dass der Schnitt in der vorgesehenen Richtung verläuft. Zum Erzeugen des Schnittes erweist sich als eine vorteilhafte Methode die Verwendung einer Säge.

In einer besonders vorteilhaften Ausgestaltung umfasst die Sägevorrichtung ein Sägeblatt, dessen Verlauf eine Sägeblattebene definiert, wobei die Sägevorrichtung mit einem Verbindungsmittel an der Referenzvorrichtung bzw. an der Verstelleinrichtung befestigt ist, und wobei das Verbindungsmittel sowie die Sägevorrichtung derart ausgestaltet ist, dass das Sägeblatt ausschliesslich in der Sägeblattebene verschiebbar gelagert ist.

Diese Ausführungsform weist den Vorteil auf, dass die Ausrichtung des Sägeblattes fest vorgegeben ist, so dass sich der Operateur ausschliesslich auf das Bewegen des Sägeblattes zum Knochen hin und auf die Durchführung der Resektion konzentrieren kann, in der Gewissheit, dass die Ausrichtung der Resektionsebene stimmt. Dies Bedeutet für den Operateur eine erhebliche Erleichterung während der Resektion, kann er sich doch im Wesentlichen auf das Schneiden konzentrieren, wobei er sich auf eventuell vorhandene Hindernisse wie Bänder konzentrieren kann, ohne sich jedoch um die Verlaufsrichtung der Säge kummern zu müssen.

Die erfindungsgemässe Vorrichtung ist motorisch angetrieben. Zudem kann ein Rechner vorgesehen sein, welcher das Verfahren der Vorrichtung sowie das Schneiden überwacht oder sogar ansteuert.

Resektionsschnitte an Femur oder Tibia werden insbesondere durchgeführt, indem eine Referenzvorrichtung am distalen Bereich des Femurs fixiert und anschliessend bezüglich der Verlaufsrichtung des Femurs ausgerichtet wird, und indem eine Schneidlehre zum Führen eines Sägeblattes oder eine Sägevorrichtung mit einem Sägeblatt mit der ausgerichteten Referenzvorrichtung verschiebbar verbunden und in einer die Verlaufsrichtung des Resektionsschnittes bestimmenden Ausrichtung geführt wird, und indem mit dem ausgerichtet geführten Sägeblatt die Resektion durchgeführt wird.

Nachfolgend wird die Erfindung anhand von Ausführungsbeispielen beschrieben. Es zeigen:
- Fig. 1a: eine perspektivische Ansicht einer auf dem Femur befestigten Basisplatte;
- Fig. 1b: eine Unteransicht einer Basisplatte;
- Fig. 2a: eine Seitenansicht einer Referenzvorrichtung;
- Fig. 2b: eine Draufsicht einer Referenzvorrichtung;
- Fig. 2c: eine Rückansicht einer Referenzvorrichtung;
- Fig. 3: eine perspektivische Ansicht einer auf dem Femur befestigten Referenzvorrichtung;
- Fig. 4: eine perspektivische Ansicht einer an der Referenzvorrichtung befestigten Kontrollehre;
- Fig. 5: eine perspektivische Ansicht eines mit der Referenzvorrichtung verbundenen Ausrichtstabes;
- Fig. 6,7: eine perspektivische Ansicht einer Tibiaschiene;
- Fig. 8: eine perspektivische Ansicht einer mit dem Ausrichtstab zu überprüfende Tibiaschiene;
- Fig. 9: eine perspektivische Ansicht eines mit einem Befestigungsbügel bei 90° flektiert gehaltenen Gelenkes;
- Fig. 10: eine an der Referenzvorrichtung befestigte Verfahreinrichtung;
- Fig. 11: eine Ansicht einer Verfahreinrichtung mit einem Basisbalken und einem daran befestigten Messbalken;
- Fig. 12: eine Ansicht einer am Basisbalken befestigten Schneidlehre;
- Fig. 13a: eine perspektivische Ansicht eines Gesamtsystems zum Einsetzen einer Knieprothese;
- Fig. 13b: eine Aufsicht auf eine Referenzvorrichtung sowie eine daran befestigte Schneidvorrichtung;
- Fig. 13c: eine Detailansicht einer Einrastvorrichtung;
- Fig. 14: eine schematische Ansicht einer mit einem Rechner angesteuerten Antriebsvorrichtung;
- Fig. 15: eine sagittale Ansicht von Femur und Tibia sowie deren Achsverläufe;
- Fig. 16a - 16d: ein weiteres Ausführungsbeispiel einer am Femur zu befestigenden Basisplatte.

Nachfolgend sind die gleichen Teile mit denselben Bezugszeichen versehen.

Ein wesentlicher Gedanke der erfindungsgemässen Vorrichtung zum Einsetzen einer Kniegelenktotalendoprothese ist die Verwendung eines Bezugssystems, welches am Femur 1 verankerbar ist. Dieses Bezugssystem dient als Referenz für alle Handhabungen und Verfahrensschritte, um die Tibia 2 bezüglich dem Femur 1 auszurichten und die Resektion an den Gelenkflächen durchzuführen. Das am Femur 1 verankerte Bezugssystem ist in einer vorteilhaften Ausgestaltung in dessen Ausrichtung bezüglich dem Femur 1 verstellbar, um das Bezugssystem insbesondere in Belastungsrichtung des Femurs 1 verlaufend auszurichten.

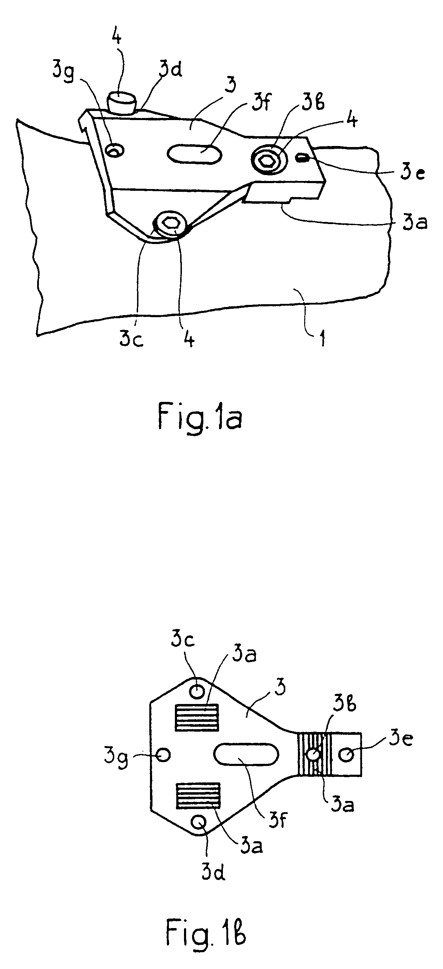

Fig. 1a zeigt eine Basisplatte 3, welche Bohrungen 3b,3c,3d aufweist zur Aufnahme von Knochenschrauben 4. Die Basisplatte 3 weist, wie auf der Unteransicht gemäss Fig. 1b ersichtlich, drei beabstandet angeordnete Auflageflächen 3a auf, welche auf dem Femur 1 zu liegen kommen, so dass die dadurch gebildete Dreipunktauflage ein verkippungsfreies Aufliegen auf dem Femur 1 gewährleistet. Die Basisplatte 3 weist zudem eine Öffnung 3f zur Aufnahme eines Bajonettverschlusses sowie zwei Ausrichtbohrungen 3e, 3g auf. Zudem weist die Bohrung 3b, wie in Fig. 1a dargestellt, eine Ansenkung auf.

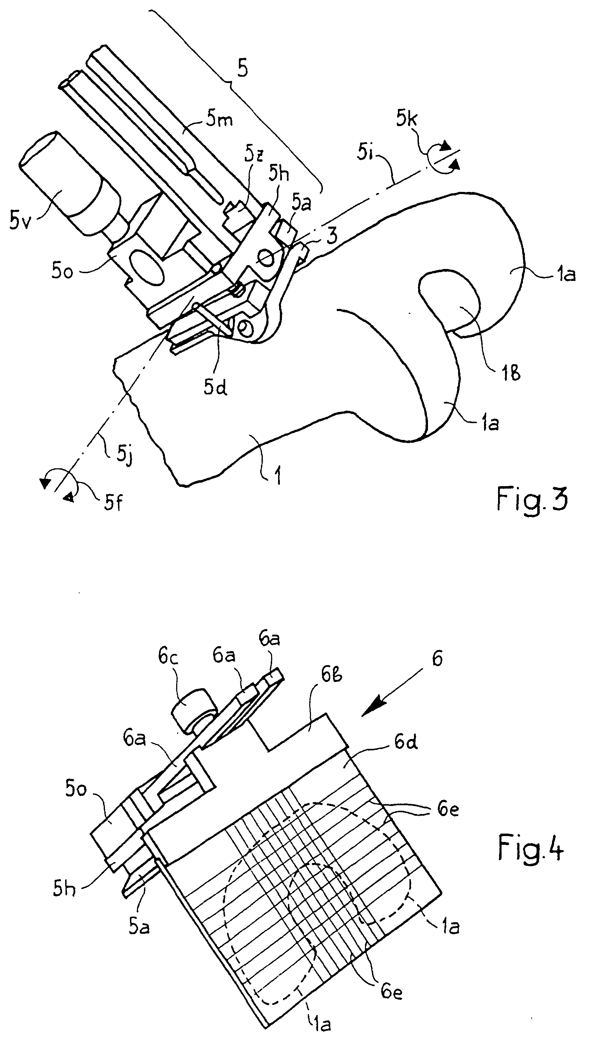

Die Basisplatte 3 wird, wie in Fig. 3 dargestellt, in der Nähe der Kondylen 1a derart auf dem Femur 1 ausgerichtet angeordnet, dass die durch die Bohrungen 3g,3e gebildete Achse vorzugsweise in Richtung der Belastungsachse 19b des Femurs 1 verläuft. Dazu werden mit einer Bohrlehre zwei Steinmannnägel ungefähr in Verlaufsrichtung der Belastungsachse 19b in den Femur 1 gesetzt und danach die Basisplatte 3 derart auf den Femur 1 aufgelegt, dass je ein Steinmannnagel durch die Bohrungen 3g,3e verläuft. Daraufhin wird eine Bohrlehre auf die Bohrungen 3b,3c,3d der Basisplatte 3 aufgesetzt, daraufhin Löcher im Femur 1 gebohrt und anschliessend Knochenschrauben 4 eingeführt, so dass die Basisplatte 3, in ihrer Längsausrichtung ungefähr in Richtung der Belastungsachse 19b verlaufend, durch die Knochenschrauben 4 mit dem Femur 1 fest verbunden ist.

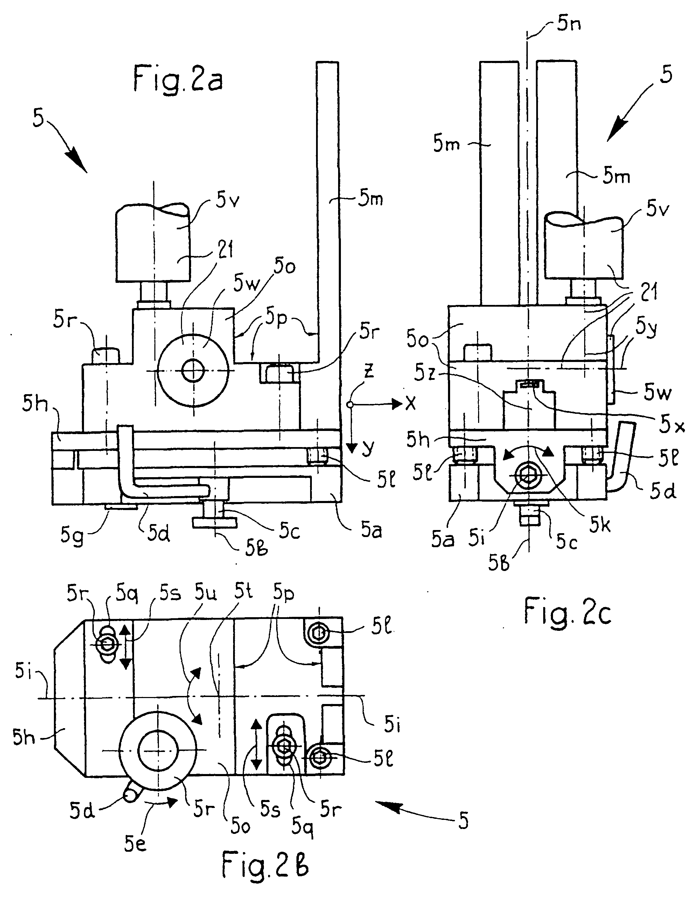

Die erfindungsgemässe Vorrichtung weist in einer bevorzugten Ausführungsform eine Referenzvorrichtung 5 auf, welche fest mit der Basisplatte 3 verbindbar ist, wobei die gegenseitige Lage von Basisplatte 3 und Referenzvorrichtung 5 einstellbar ist, um den Verlauf der Resektionslinien an Femur und Tibia möglichst exakt einzustellen. Die Figuren 2a bis 2c zeigen eine derartige Referenzvorrichtung 5, welches Teilelemente aufweist, deren Ausrichtung ein Koordinatensystem X,Y,Z definieren, bezüglich welchem Koordinatensystem alle weiteren Manipulationen und Schnitte an Femur 1 und Tibia 2 vorgenommen werden.

Die Referenzvorrichtung 5 umfasst ein Basisteil 5a, an welchem ein Verschlussteil 5c eines Bajonettverschlusses mit Drehachse 5b und Betätigungshebel 5d angeordnet ist. Das Basisteil 5a wird derart mit der Basisplatte 3 verbunden, dass der Hebel 5d in die dargestellte Position gebracht wird, daraufhin das Verschlussteil 5c in die Öffnung 3f eingeführt wird, und daraufhin das Einrastteil 5g in die Ansenkung der Bohrung 3b eingeführt wird.. Daraufhin wird der Betätigungshebel 5d in Richtung 5e bewegt, so dass der durch die Teile 5c,3f gebildete Bajonettverschluss einrastet und das Basisteil 5a fest jedoch lösbar mit der Basisplatte 3 verbunden ist.

Auf dem Basisteil 5a ist eine um die Schwenkachse 5i in Richtung 5k schwenkbar gelagerte Schwenkplatte 5h angeordnet, wobei die Schwenkplatte 5h zwei Bohrungen mit Innengewinde zur Aufnahme je einer Inbusschraube 51 aufweist. Diese Schrauben 51 werden derart tief in das Innengewinde gedreht, dass sie auf dem Basisteil 5a aufliegen. Die relative Neigung zwischen dem Basisteil 5a und der Schwenkplatte 5h lässt sich, wie am besten aus Fig. 2c ersichtlich, durch die jeweilige Einschraubtiefe der beiden gegenüberliegend angeordneten Inbusschrauben 51 einstellen.

Ein Referenzkörper 5o aufweisend Längsbohrungen 5q ist mittels Schrauben 5r fest mit der Schwenkplatte 5h verbindbar. Die Längsbohrung 5q, auch als Langloch bezeichnet, ist breiter als der Schaft der Schraube 5r ausgestaltet. Bei gelösten Schrauben 5r ist der Referenzkörper 5o auf Grund der Längenausdehnung der Längsbohrungen 5q in Bewegungsrichtung 5s entweder parallel verschiebbar oder zudem auch um die Drehachse 5t in Bewegungsrichtung 5u verschiebbar. Somit lässt sich der Referenzkörper 5o bezüglich der Schwenkplatte 5h verschiebbar, und insbesondere leicht versetzt anordnen, und mit Hilfe der Schrauben 5r fest verbinden. Der Referenzkörper 5o definiert über die Referenzflächen 5p sowie die fest mit dem Referenzkörper 5o verbundenen Gabeln 5m die Ausrichtung des Koordinatensystems X,Y,Z, welches das Referenz-Koordinatensystem ausbildet. Im Referenzkörper 5o ist, wie aus Fig. 2c ersichtlich, eine Führungsöffnung 5z angeordnet, welche eine in X-Richtung verlaufende Längsführung für eine Zahnstange 10a ausbildet. Der Referenzkörper 5o weist ein in seinem Inneren angeordnetes Schneckengetriebe 5w auf, welche zwei senkrecht verlaufende Drehachsen 5y umfasst, wobei an der einen Drehachse 5y eine Rändelschraube 5v sowie innerhalb des Referenzkörpers 50 eine Schnecke angeordnet ist, und an der anderen Drehachse 5y ein Getriebe 5w sowie ein in die Längsführung 5z vorstehendes Zahnrad 5x, welches zum Eingriff in die Zahnstange 10a bestimmt ist. Das Zahnrad 5x könnte auch direkt auf einer Achse 5y der Rändelschraube 5v befestigt sein, so dass auch ein Schneckengetriebe 5w verzichtet werden könnte.

Fig. 3 zeigt einen Femur 1, auf welchem die Basisplatte 3 angeschraubt ist. Die Referenzvorrichtung 5 ist mit der Basisplatte 3 verbunden und kann durch ein Betätigen des Betätigungshebels 5d jederzeit gelöst und entfernt oder wieder befestigt werden. Aus Fig. 3 ist zudem die in X-Richtung verlaufende Längsführung 5z ersichtlich. In einer weiteren Ausführungsvariante könnte die Referenzvorrichtung 5 auch derart ausgestaltet sein, dass diese zusätzlich zur Schwenkachse 5i in einer zur Schwenkachse 5i senkrecht verlaufenden zweiten Schwenkachse 5j bezüglich dem Basisteil 5a in Bewegungsrichtung 5f schwenkbar gelagert ist, wobei der Schwenkwinkel wiederum durch Schrauben fest einstellbar und fixierbar ist. Die Referenzvorrichtung 5 könnte auch, unter Verzicht der Schwenkachse 5i, einzig eine Schwenkachse 5j aufweisen.

Fig. 4 zeigt symbolisch den Referenzkörper 5o, in dessen Längsführung 5z ein Einsteck- und Halteteil 6a einer Kontrollehre 6 eingelassen ist. Die Kontrollehre 6 umfasst einen Halter 6b mit einem daran befestigten, transparenten Körper 6d mit Gitterlinien 6e. Die Kontrollehre 6 dient zum Ausrichten der Schwenkplatte 5h in Schwenkrichtung 5k. Dazu wird in der Anordnung gemäss Fig. 3 das Einsteckteil 6a in die Längsführung 5z eingesteckt und danach der in Verlaufsrichtung des Halteteils 6a beziehungsweise in X-Richtung verschieblich gelagerte transparente Körper 6d mit Halter 6b derart verschoben, dass der transparente Körper 6d, wie in Fig. 4 angedeutet, unmittelbar vor die Femurkondyle 1a zu liegen kommt. Daraufhin wird der Halter 6b mit der Rändelschraube 6c am Halteteil 6a fixiert. Die Gitterlinien 6e verlaufen dabei bezüglich dem durch den Referenzkörper 5o vorgegebenen Koordinatensystem in Y- und Z-Richtung. Durch entsprechendes Drehen der Schrauben 51 kann der transparente Körper 6d um die Schwenkachse 5i drehend verstellt werden. Zudem kann die Lage des transparenten Körper 6d durch verschieben des Referenzkörpers 5o in Richtung 5s bzw. in Richtung 5u eingestellt werden. Somit kann die Lage des Referenzkörpers 5o beziehungsweise das Koordinatensystem in Y- und Z-Richtung bezüglich der Lage der Kondylen 1a äusserst genau eingestellt werden.

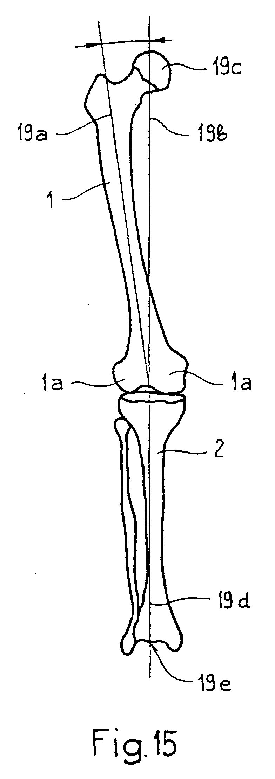

Die Belastungsachse 19b (Weight Bearing Axis WBA) des Femurs 1 verläuft bekannterweise, wie in Fig. 15 dargestellt, durch das Zentrum des Hüftkopfes 19c sowie durch das Sprunggelenkzentrum 19e. Die anatomische Achse 19a des Femurs 1 ist gegenüber dieser Belastungsachse 19b geneigt. Der Verlauf der Tibia 2 definiert eine mechanische Achse 19d. In der dargestellten Lage weisen der Femur 1 sowie die Tibia 2 eine Flexion von 0° auf und die Belastungsachse 19b und die mechanische Achse 19d verlaufen deckungsgleich.

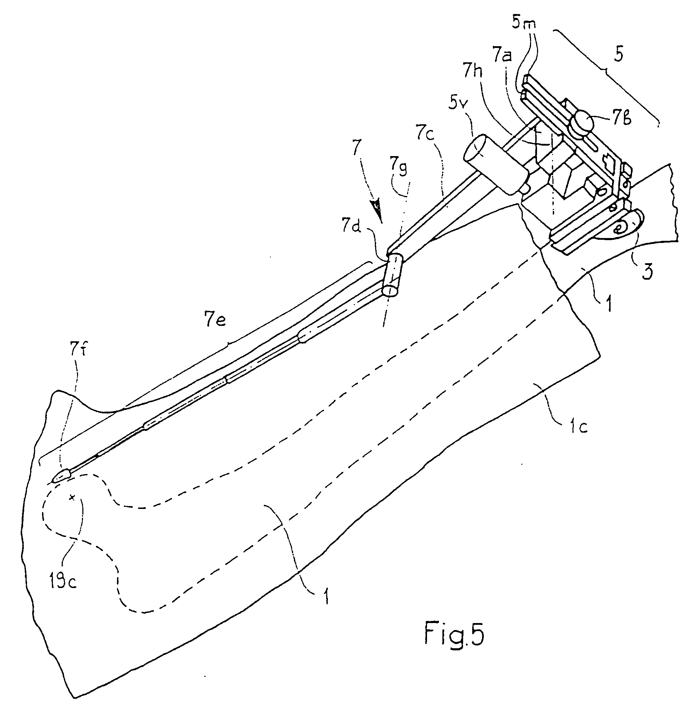

Fig. 5 zeigt einen Ausrichtstab 7, welcher den Referenzkörper 5o bezüglich der Lage des Hüftkopfes 19c auszurichten erlaubt. Der Ausrichtstab 7 umfasse einen Befestigungsblock 7a, welcher mittels einer Rändelschraube 7b an der Gabel 5m befestigbar ist. Ein Teleskopstab 7e mit Endzeiger 7f ist über das Gelenk 7d mit Drehachse 7g und den Bügel 7c mit Drehachse 7h am Befestigungsblock 7a gelagert. Der Ausrichtstab 7 ist derart ausgestaltet und an der Referenzvorrichtung 5 angeordnet, dass der Teleskopstab 7e im wesentlichen oder möglichst genau in X-Richtung verläuft, und in der XY-Ebene schwenkbar gelagert ist. Die Lage des Referenzkörpers 5o wird mit gelösten Schrauben 5r derart eingestellt, dass durch Palpieren, beispielsweise mit der sogenannten "Zweifingermethode", das Zentrum des Hüftkopfes 19c ertastet wird, und danach der Endzeiger 7f des Teleskopstabes 7e am Oberschenkel 1c an diese Stelle angelegt wird, wodurch der Referenzkörper 5o derart ausgerichtet wird, dass die Projektion der X-Achse (in sagittaler Richtung) durch das Zentrum des Hüftkopfes 19c verläuft. Zudem kann zusätzlich mit Hilfe der Gitterlinien 6e der Verlauf der X-Achse derart eingestellt werden, dass die X-Achse durch die Mitte der Kondyle 1a verläuft. Somit verläuft die X-Achse, aus einer sagittalen Ansicht gemäss Fig. 15, deckungsgleich zur Belastungsachse 19b.Die Schrauben 5r werden angezogen und dadurch die Lage des Referenzkörpers 5o bezüglich der Schwenkplatte 5h fixiert. Somit ist durch die Ausrichtung der Lage des Referenzkörpers 5o das Bezugssystem bzw. die orthogonalen Achsen in X-, Y- und Z-Richtung definiert festgelegt.

Die Referenzvorrichtung 5 weist den Vorteil auf, dass alle getätigten Einstellungen an der Referenzvorrichtung 5 vorgenommen wurden und somit quasi auf dieser gespeichert sind. Daher ist es möglich die derart eingestellte Referenzvorrichtung 5 über den Bajonettverschluss 5b von der Basisplatte 3 zu lösen und zu entfernen, um an Femur 1 oder Tibia 2 weitere Manipulationen vorzunehmen. Zu einem späteren Zeitpunkt kann die Referenzvorrichtung 5 wieder auf der Basisplatte 3 befestigt werden, wobei die Achsen in X-, Y- und Z-Richtung wie bereits vorgängig definiert verlaufen und daher nicht mehr eingestellt werden müssen.

Ein Vorteil der Referenzvorrichtung 5 ist darin zu sehen, dass sich der Verlauf der Achsen X,Y und Z bezüglich der Lage des Femurs 1 sowie der Kondylen 1a sehr genau einstellen lassen. Die Referenzvorrichtung 5 könnte auch derart einfacher ausgebildet sein, dass eine Verstellung des Referenzkörpers 5o bezüglich der Basisplatte 3 nur in einer oder in zwei Dimensionen möglich ist.

Falls die Trennbarkeit zwischen Basisplatte 3 und Referenzvorrichtung 5 nicht notwendig ist, kann in einer weiteren, einfacheren Ausführungsform auf die Basisplatte 3 verzichtet werden, in dem das Basisteil 5a der Referenzvorrichtung 5 direkt am Femur 1 angeschraubt wird.

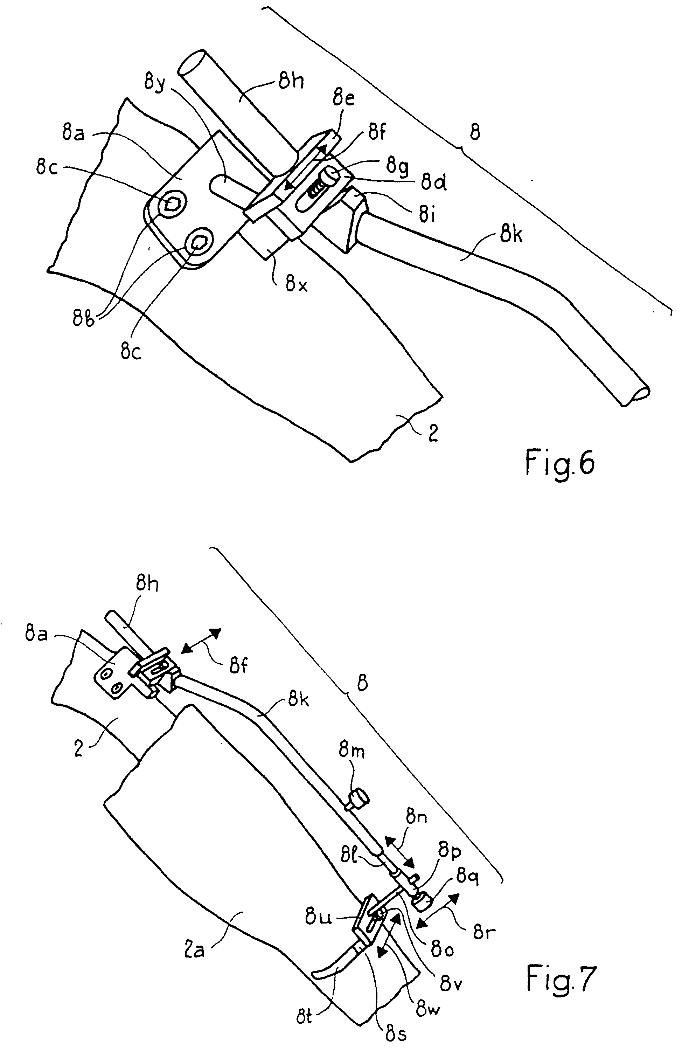

Zum Ausrichten der Tibia 2 ist eine Tibiaschiene 8 vorgesehen, welche in den Figuren 6 und 7 dargestellt ist. Die Tibiaschiene 8 wird vorzugsweise derart an der Tibia 2 befestigt, dass die Tibiaschiene 8, in einer sagittalen Ansicht gemäss Fig. 15, deckungsgleich zur mechanischen Achse 19d der Tibia 2 verläuft. Eine Tibiaplatte 8a aufweisend zwei Bohrungen 8b wird mit zwei Knochenschrauben 8c an der Tibia 2 verankert. Ein Auflageteil 8d ist über ein arretierbares Kugelgelenk 8x und das Verbindungsteil 8y mit der Tibiaplatte 8a verbunden. Das Kugelgelenk 8x ist innerhalb des mit 8x bezeichneten Körpers angeordnet. Ein Tibiastab 8k mündet über ein Anschlagteil 8i und einen Anschlag 8e in einen Endabschnitt 8h. Der Tibiastab 8k ist in gelöstem Zustand in Verschieberichtung 8f verschiebbar, wobei mit dem Auflagesteg 8d eine Schraube 8g verbunden ist, welche in angezogenem Zustand den Tibiastab 8k fest mit dem Körper 8x fixiert, wodurch die Lage des Tibiastabes 8k in Verschieberichtung 8f fixiert ist. Am dem Kniegelenk entfernten Ende ist ein Auflageteil 8s mit Auflage 8t am Unterschenkel 2a angelegt und mit diesem beispielsweise mit Hilfe von Binden fixiert. Ein Verschiebeteil 8u ist bezüglich dem Auflageteil 8s in Verschieberichtung 8w verschiebbar und mit einer Schraube 8v fixierbar. Der Tibiastab 8k mündet in einen Verschiebestab 81, welcher bezüglich der Längsrichtung 8n des Tibiastabes 8k verschiebbar gelagert und mit Hilfe einer Rändelschraube 8m mit dem Tibiastab 8k fixierbar ist. Am Verschiebeteil 8u ist ein stabförmiger Halter 8o-vorstehend angeordnet, welcher in ein Führungsteil 8p der Verschiebestabes 81 einführbar, in Verschieberichtung 8r verstellbar, und mit einer Rändelschraube 8q fixierbar ist. Die beschriebenen Verstellmöglichkeiten des Tibiastabes 8k bezüglich der Tibiaplatte 8a sowie dem Auflageteil 8s erlauben dessen Verlauf derart einzustellen, dass der Tibiastab 8k in sagittaler Richtung deckungsgleich zur anatomischen Achse 19d der Tibia 2 verläuft.

Spätestens nach dem Anbringen der Tibiaschiene 8 wird ein Spanninstrument 20, von welchem eine Ausführungsbeispiel in der Druckschrift FR 2 648 699 offenbart ist, zwischen dem Femur 1 und der Tibia 2 eingebracht.

Das Spanninstrument 20 basiert auf dem Prinzip einer Spreizzange und dient dazu, die Gelenkflächen der Tibia 2 und des Femur 1 einzeln an medialem bzw. lateralem Kondyl so auseinanderzudrücken, dass die gewünschte Ausrichtung zwischen Femur 1 und Tibia 2 entsteht. Mit der erfindungsgemässen Vorrichtung lässt sich die Anforderung, dass die Belastungsachse 19b des Femurs 1 in Richtung der mechanischen Achse 19d der Tibia 2 verläuft auf einfache Weise dadurch erzielen, dass der den Verlauf der Belastungsachse 19b anzeigende Ausrichtstab 7 um die Drehachse 7h drehend zur Tibia 2 hin geschwenkt wird. Da der Tibiastab 8k den Verlauf der mechanischen Achse 19d der Tibia 2 anzeigt kann nunmehr die Tibia 2 durch ein entsprechendes Verstellen der Spannvorrichtung 20 derart ausgerichtet werden, dass der Tibiastab 8k sagittal deckungsgleich bezüglich dem Ausrichtstab 7 verläuft. Durch ein Verstellen an der Spannvorrichtung 20 kann die Beinachse in varus-valgus-Richtung auch leicht abgewinkelt verlaufend eingestellt werden. Somit besteht die Möglichkeit beabsichtigt einen Winkel zwischen der Belastungsachse 19b und der Tibiaachse 19d einzuführen.

Lassen die medialen und lateralen Bänder die für die gewünschte Beinachsenstellung erforderlichen Aufspreizungen nicht zu, müssen durch bekannte chirurgische Eingriffe die Spannungen der Bänder durch teilweises Trennen an den Anwachsstellen des Knochens gelöst werden. Dies hat so lange zu geschehen bis in allen Flexionsstellungen geeignete Bänderspannungen existieren.

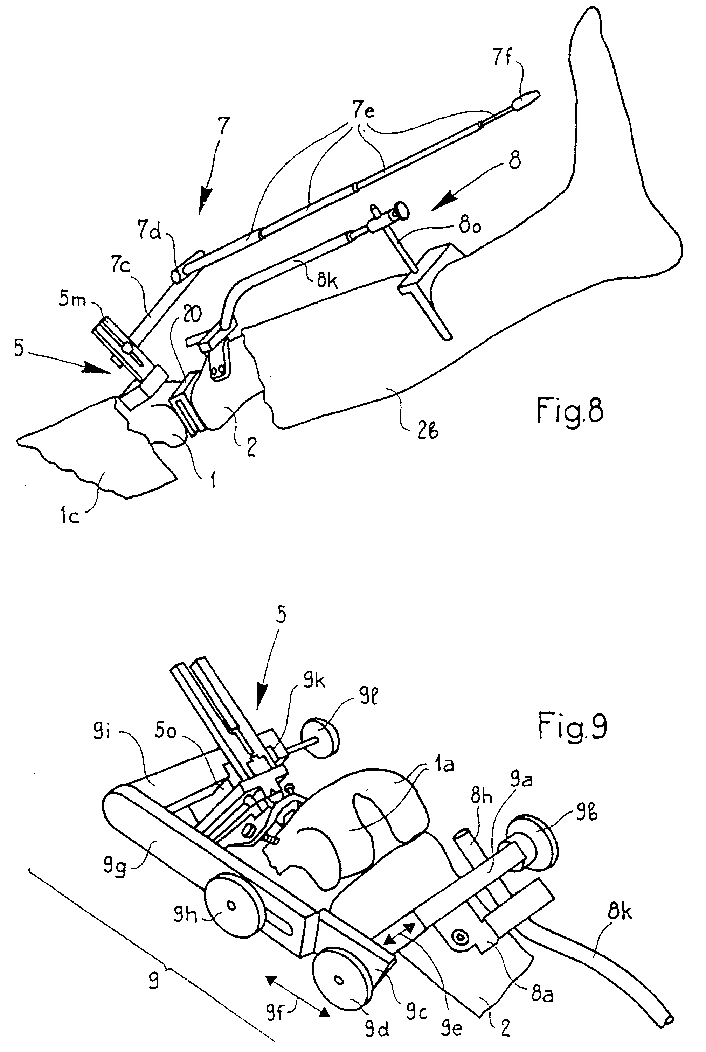

Danach wird das Gelenk bei eingesetzter Spannvorrichtung 20 in eine 90° flektierte Lage gebracht, wie dies in Fig. 9 dargestellt ist. Dabei liegt der Oberschenkel sowie der Unterschenkel auf einer entsprechenden Stütze auf. Daraufhin werden der Femur 1 und die Tibia 2 in dieser 90° flektierten Lage mit Hilfe eines U-förmigen Befestigungsbügel 9 gegenseitig fixiert. Der Befestigungsbügel 9 umfasst einen Tibiaschienenhalter 9a über dessen Rändelschraube 9b der Endabschnitt 8h des Tibiastabes 8k fest einspannbar ist. Der Befestigungsbügel 9 umfasst weiter ein Bügelgrundteil 9g sowie ein Bügelverstellteil 9c, welches bezüglich dem Bügelgrundteil 9g in Längsrichtung 9f verstellbar und mit einer Rändelschraube 9h fixierbar ist. Der Tibiaschienenhalter 9a ist in Schieberichtung 9e schiebbar mit dem Bügelverstellteil 9c verbunden und mit der Rändelschraube 9d fixierbar. Der Bügelgrundteil 9g ist fest mit einem Bügelquerteil 9i verbunden, welches eine Ausnehmung 9k zur Auflage auf dem Referenzkörper 5o aufweist. Der Bügelquerteil 9i ist über eine Rändelschraube 91 fest mit dem Referenzkörper 5o verbindbar. Die Mehrzahl an Verstellmöglichkeiten des Befestigungsbügels 9 erlauben es den Endabschnitt 8h des Tibiastabes 8k in der vorgegebenen Lage fest mit dem Referenzkörper 5o zu verbinden. Nach diesem Schritt sind der Femur 1 und die Tibia 2 in einer genau definierten Lage gegenseitig fest gehalten. Bevorzugt fluchten in dieser Lage die Belastungsachse 19b sowie die Tibiaachse 19d gegenseitig, und deren aufgespannter Winkel beträgt 90 Grad. In einer weiteren vorteilhaften Ausführungsform könnte der Befestigungsbügel 9 auch rechteckförmig ausgestaltet sein, in dem der Tibiaschienenhalter 9a sowie das Bügelquerteil 9i beidseitig mit je einem Bügelgrundteil 9g und einem Bügelverstellteil 9c verbunden sind. Ein derartiger, rechteckförmiger Befestigungsbügel 9 weist gegenüber einer Ausführungsform gemäss Fig. 9 eine erhöhte Stabilität auf.

Daraufhin wird die Spannvorrichtung 20 entfernt und der Femur 1 und die Tibia 2 sind, wie in Fig. 9 dargestellt, gehalten. Der erfindungsgemässe Befestigungsbügel 9 weist den Vorteil auf, dass der Femur 1 und die Tibia 2 in einer definiert ausgerichteten Lage fest gehalten sind, und dass durch die U-förmige Ausgestaltung des Befestigungsbügels 9 der Zugang zum Operationsfeld nicht behindert ist.

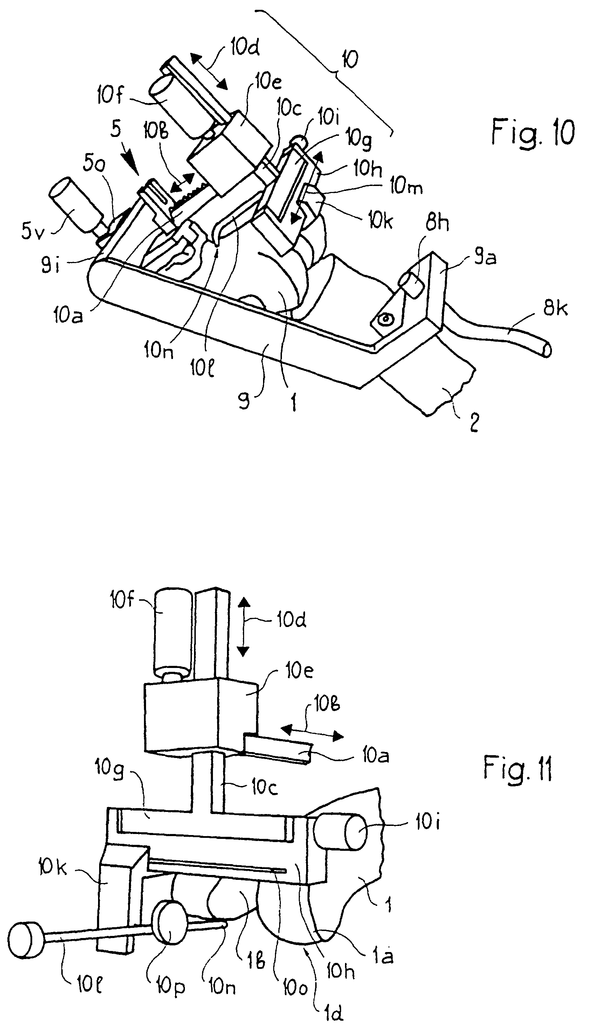

Fig. 10 zeigt die Anordnung gemäss Fig. 9, wobei zusätzlich an der Referenzvorrichtung 5 eine Verfahreinrichtung 10 angeordnet ist, welche einen Basisbalken 10g und daran befestigte Adapterteile 10h in X- und Y-Richtung zu bewegen erlaubt. Die Verfahreinrichtung 10, auch als eine Verstelleinrichtung oder eine Vorschubeinrichtung zu bezeichnen, umfasst eine vorschubeinrichtung 10e, welche mit einer Zahnstange 10a fest verbunden ist. Diese Zahnstange 10a ist teilweise in der Längsführung 5z verlaufend angeordnet, wobei das Zahnrad 5x der Rändelschraube 5v in die Zahnstange 10a eingreift, um die Zahnstange 10a in Verschieberichtung 10b, welche der X-Richtung entspricht, zu bewegen. Die Vorschubeinrichtung 10e weist, analog der in den Figuren 2a bis 2c dargestellte Referenzvorrichtung 5, eine Rändelschraube 10f auf, welche ein nicht sichtbares Schneckengetriebe antreibt, das über ein in einer Längsführung angeordnetes Zahnrad in eine durch die Längsführung geführte Zahnstange 10c eingreift, um diese in Verschieberichtung 10d zu bewegen. Im Ausführungsbeispiel gemäss Fig. 10 ist die Verschieberichtung 10d identisch zur Y-Richtung. Die beiden Bewegungsachsen bzw. Verschieberichtungen 10b,10d verlaufen vorzugsweise rechtwinklig zueinander, können jedoch auch unter einem anderen Winkel zueinander verlaufen. Vorteilhafterweise verläuft die eine Verschieberichtung 10b parallel zur Belastungsachse 19b, wogegen die zweite Verschieberichtung 10d senkrecht zur Belastungsachse 19b verläuft. Statt an der Referenzvorrichtung 5 könnte die Verstelleinrichtung 10 auch am Befestigungsbügel 9 angeordnet sein, in dem der Befestigungsbügel 9 eine Längsführung 5z sowie eine Rändelschraube 5v zum Aufnehmen und Bewegen der Zahnstange 10a aufweist.

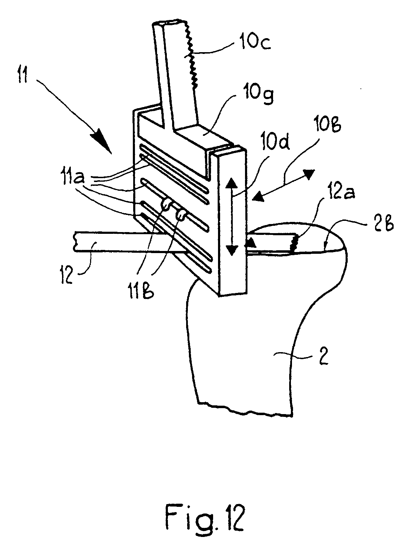

Um die Resektion des Femurs und der Tibia durchzuführen wird, wie in Fig. 12 dargestellt, am Basisbalken 10g der Verstelleinrichtung 10 eine Schneidlehre 11 befestigt, welche in unterschiedlichen Winkeln verlaufende Schlitze 11a aufweist, um das Sägeblatt 12 mit Sägezähnen 12a exakt in den durch das Implantat vorbestimmten Winkeln zu führen. Fig. 12 zeigt einen mit dem Sägeblatt 12 durchgeführten Resektionsschnitt an der Tibiafront 2b. Je nach Ausführungsform einer Kniegelenkendoprothese können die Winkel der Resektionsschnitte unterschiedlich verlaufen. Daher sind unterschiedliche Schneidlehren 11 verfügbar, wobei die jeweils geeignete Schneidlehre 11 am Basisbalken 10g befestigt wird. Die Schneidlehre 11 weist zudem Bohrungen 11b zum Führen eines Bohrers für den Patellakanal auf. Die Schneidlehre 11 kann durch manuelles Drehen an den Rändelschrauben 5v, 10f in die erforderliche Stelle gefahren werden. Dabei wird die Schneidlehre 11 exakt parallel verfahren, so dass exakt parallel verlaufende Resektionsflächen an Femur 1 und Tibia 2 erstellbar sind. Die Zahnstangen 10a, 10c könnten einen Massstab aufweisen, zum Beispiel einen an der Oberfläche eingravierten Massstab, über welchen der verfahrene Weg ablesbar ist. Dies ist insbesondere bei einem manuellen Verfahren bzw. einem manuellen betätigen der Rändelschrauben 5v,10f von Vorteil. Die Möglichkeit eines manuellen Verfahrens weist den Vorteil auf, dass die Vorrichtung selbst beim Ausfall eines Computers oder eines Motors noch betätigbar ist, so dass auch in einer derartige Notsituation ein Weiterführen der Operation gewährleistet ist.

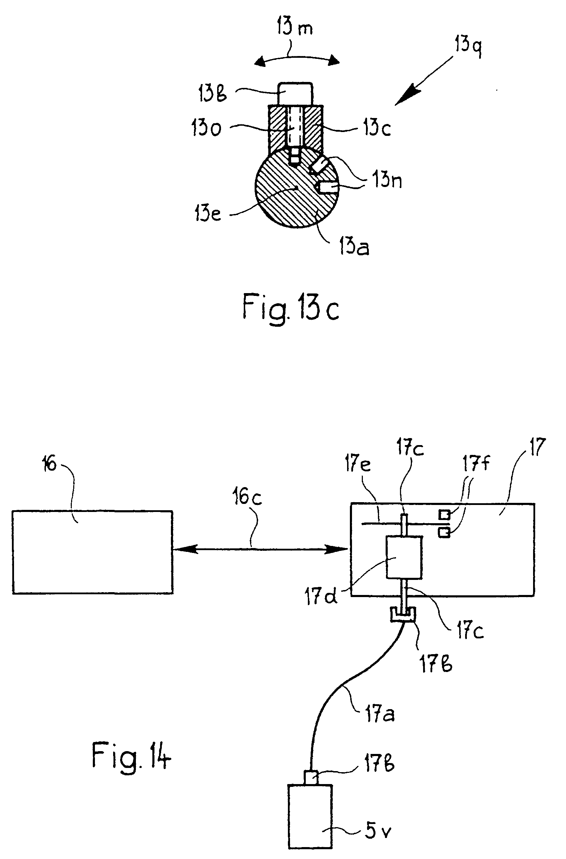

Die Rändelschrauben 5v, 10f sind motorisch angetrieben. Fig. 14 zeigt schematisch eine erfindungsgemässe Antriebsvorrichtung 17, welche über eine bidirektionale Datenleitung 16c mit einem Rechner 16 verbunden und von diesem angesteuert ist. Die Antriebsvorrichtung 17 umfasst einen Elektromotor 17d mit einer Welle 17c. An dieser Welle 17c ist eine Winkelscheibe 17e sowie ein Sensor 17f zum Erfassen des Drehwinkels angeordnet. Der Elektromotor 17d wird vom Rechner 16 angesteuert und der Drehwinkel des Elektromotors 17d über das Sensorsignal 17f vom Rechner 16 überwacht. Die Rändelschraube 5v, 10f und die Welle 17c sind über eine flexible Welle 17a, welche beiden Endes ein Adapterteil 17b aufweist, miteinander verbunden. Die flexible Welle 17a ist vorzugsweise aus einem Metalldraht ausgestaltet. Die Anordnung gemäss Fig. 14 weist die folgenden Vorteile auf:
Operationen am Knochen stellen höchste Anforderungen bezüglich Sterilität. Daher müssen alle Objekte, die nahe beim Operationsfeld sind, sterile Eigenschaften aufweisen. Es wäre mit erheblichem Aufwand verbunden einen sterilisierbaren Elektromotor zu bauen, welcher direkt an der Verfahrvorrichtung 10 angeordnet werden könnte. Die Verwendung eines Metalldrahtes, z.B. Federstahldraht, weist den Vorteil auf, dass der Elektromotor zum Beispiel ein bis zwei Meter entfernt vom Operationsfeld angeordnet werden kann. Insbesondere die Verwendung einer Federstahldrahtsaite weist den Vorteil eines hohen Elastizitätsmoduls sowie einem geringen Hystereseeffekt auf. Durch den grösseren Abstand zum Operationsfeld bestehen bezüglich der Sterilität der Antriebsvorrichtung 17 geringere Anforderungen. Die erfindungsgemässe Welle 17a weist zudem den Vorteil auf, dass sie sterilisierbar ist, und, da sie günstig herstellbar ist, auch als Einwegprodukt konzipierbar ist. Die Antriebsvorrichtung 17 weist zudem den Vorteil auf, dass die Rändelschraube 5v sowohl antreibbar ist, als auch deren Drehwinkel über den Sensor 17f überwachbar ist. Die Antriebsvorrichtung 17 kann auch mehrere unabhängige Antriebe für biegsame Wellen 17a aufweisen. Die Welle 17a kann als ein Volldraht oder auch als ein Hohldraht ausgestaltet sein. Vorzugsweise werden Stahldrähte verwendet, wobei auch Drähte andere Metalle oder aus Kunststoff oder Verbundmaterial geeignet sind. In einer weiteren, vorteilhaften Ausführungsform könnte die Antriebsvorrichtung 17 mit Elektromotor 17d, Winkelscheibe 17e sowie Sensor 17f in oder an Stelle der Rändelschraube 5v angeordnet sein, wobei die Antriebsvorrichtung 17 über eine elektrische Ansteuer- und Datenleitung 16c mit einem Rechner 16 verbunden ist.

In einer bevorzugten Ausführungsform wird die Verfahreinrichtung 10 gemäss Fig. 10 mit einer Antriebsvorrichtung 17 gemäss Fig. 14 angetrieben, in dem die Rändelschrauben 15v, 10f mit je einer Welle 17a verbunden sind. Damit ist es nicht nur möglich den Basisbalken 10g mit daran befestigtem Adapterteil 10h in X- und Y-Richtung zu verfahren, sondern zudem die Geometrie der Kondyle 1a an ausgewählten Punkten wie auch das Tibiaplateau zu vermessen. Im Ausführungsbeispiel gemäss Fig. 10 ist am Adapterteil 10h eine Führung 10k für einen Messtaster 101 mit Messspitze 10n angeordnet. Die Führung 10k ist in Richtung 10m verschieblich am Adapterteil 10h gelagert.

Die Geometrie einer Femurkondyle 1a kann beispielsweise wie folgt vermessen werden:
Der Basisbalken 10g wird vorerst ohne ein daran befestigtes Adapterteil 10h derart verfahren, dass der Basisbalken 10g an der Front des Femurs 1 mit der Femurkondyle 1a in Anschlag gelangt. Dies blockiert das Drehen der Welle 17b, was vom Sensor 17f erfassbar ist. Somit kann die Lage der Front des Femurs 1 bestimmt und vom Rechner 16 gespeichert werden. Daraufhin wird der Basisbalken 10g wieder weggefahren und am Basisbalken 10g, wie in Fig. 10 dargestellt, der Adapterteil 10h mit Messtaster 101 angeordnet. Daraufhin wird der Basisbalken 10g verfahren bis die Tasterspitze 10n des Messtasters 101 in die dargestellte Auflage mit dem Femur 1 gelangt. Diese Position wird vom Rechner 16 gespeichert. Daraufhin wird, wie in Fig. 11 dargestellt, der Basisbalken 10g wieder weggefahren, und der Führung 10k für den Messtaster ein weiterer Messtaster 101 angeordnet, welcher mit einer Rändelschraube 10p befestigbar ist. Wird der Messtaster 101 exzentrisch angeordnet, so kann durch das Verfahren des Basisbalkens 10g die dorsale Ausdehnung 1d der Kondyle 1a vermessen werden. Wird der Messtaster 101 zentrisch angeordnet, so kann durch das Verfahren des Basisbalkens 10g die Tiefe der Grube 10b ausgemessen werden. Die Kondyle kann auch an mehreren Punkten vermessen werden durch eine entsprechende Ausgestaltung des Messtasters. Der Messtaster 101 gemäss Fig. 11 könnte auch dazu dienen die gesamte Breite der Kondylen 1a zu messen in dem die Tastspitze 10n medial und lateral mit den Kondylen 1a in den Anschlag gebracht wird, wobei vorteilhafterweise ein am Adapterteil 10h in dessen Längsrichtung verlaufender Massstab angeordnet ist, welcher die seitliche Lage der Tastspitze abzulesen erlaubt, so dass auf Grund der gemessenen, medialen und lateralen Ausdehnung der Kondylen 1a die Gesamtbreite der Femurgelenkkopfes bestimmt werden kann. Diese Breite kann in den Rechner zum Beispiel von Hand eingegeben werden, so dass dem Rechner die geometrischen Daten des Femurgelenkkopfes für weitere Berechnungen zur Verfügung stehen.

Die Messtaster 101 können auf unterschiedlichste Weise ausgeformt sein, um unter Berücksichtigung der anatomischen Form des Femurs dessen Oberfläche abzustasten. So könnte ein Messtaster 101 auch derart ausgestaltet sein, dass er, ähnlich wie in Fig. 11 dargestellt, an der Führung 10k angeordnet den dorsalen Bereich des Femurs 1 abzutasten erlaubt.

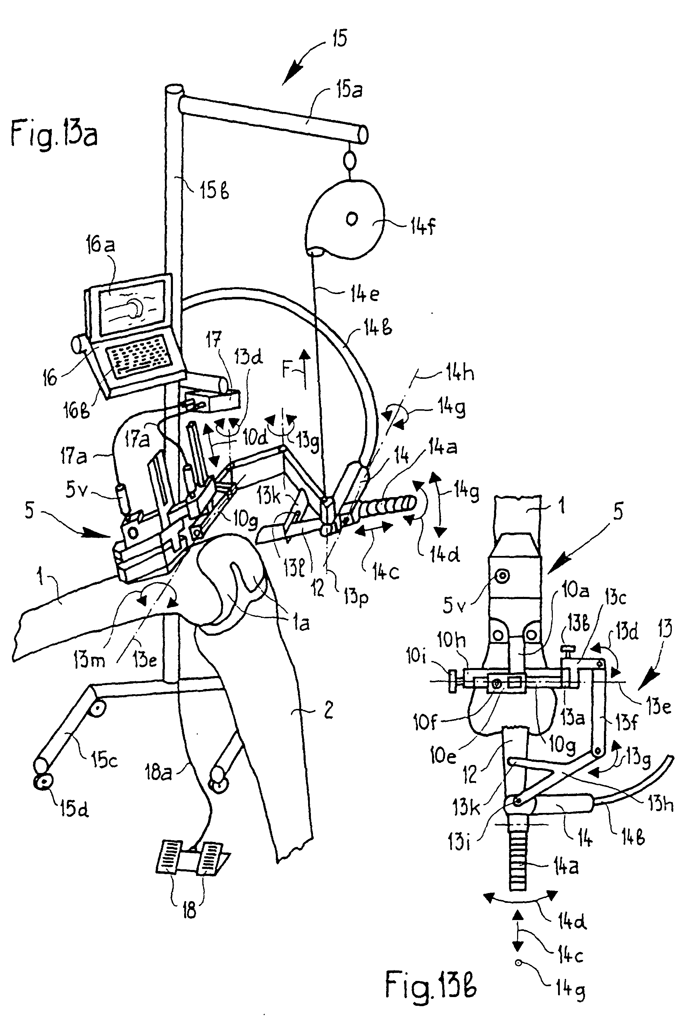

Das Gesamtsystem zum Implantieren eines Kniegelenktotalendoprothese umfasst in einer bevorzugten Ausführungsform einen Rechner bzw. einen Computer mit einem Bildschirm. Die Koordinaten der Lage der mit den Messtastern 101 ermittelten Messpunkte der Kondyle des Femurs werden über die Antriebsvorrichtung 17 dem Rechner übermittelt, wobei dem Rechner sowohl die Teilung des Winkelrades 17e als auch das Übersetzungsverhältnis des Getriebes der Vorschubeinheit 10e vorgegeben ist, so dass der Rechner die Abstände der einzelnen Messpunkte in absoluten Koordinaten und vorzugsweise in der Einheit Millimeter berechnen kann. Im Rechner ist zudem eine Datenbank mit den geometrischen Daten verfügbarer Kniegelenkimplantate abgespeichert, wobei der Rechner diese Daten mit den gemessenen Daten vergleicht und ein optimal passendes Kniegelenkimplantat vorschlägt und dies auf dem Bildschirm darstellt. In einer bevorzugten Ausführungsform wird am Bildschirm, wie in Fig. 13a dargestellt, der ausgemessene Femur, die Resektionslinien sowie das am Femur anzubringende Kniegelenkimplantat dargestellt. Der Chirurge überprüft den dargestellten Vorschlag und bestätigt entweder diesen Vorschlag, verschiebt die Resektionslinien in ihrer Gesamtheit, oder wählt ein anderes, ihm besser geeignet erscheinendes Kniegelenkimplantat aus. Nach der Auswahl des geeigneten Kniegelenkimplantates greift der Rechner auf eine Datenbank zu, in welcher alle geometrischen Daten des Implantates, insbesondere auch die Anordnung und der Verlauf der Normanlageflächen des Implantates bzw. die entsprechenden Resektionslinien gespeichert sind. Basierend auf diesen Daten bestimmt der Rechner welche aus einer Mehrzahl verfügbarer Schnittlehren 11 am Basisbalken 11g zu befestigen ist, um die vorher bestimmten Schnitte zu tätigen. An sich könnte nur eine einzige Schnittlehre 11 vorgesehen sein, welche die Winkel der jeweiligen Resektionsschnittlinien bestimmt. Durch das Bereitstellen unterschiedlicher Schnittlehren 11, deren Schnittlinienverlauf auf entsprechende Implantate abgestimmt sind, können die Resektionslinien am Femur entsprechend der Ausgestaltung sowie Grösse eines jeweiligen Implantates geschnitten werden. Nach dem Befestigen der Schnittlehre 11 wird die Verfahreinrichtung 10 vom Rechner derart angesteuert, dass die Schnittlehre 11 in die erste Schneidposition gefahren wird. Daraufhin kann der Chirurge, wie in Fig. 12 dargestellt, das Sägeblatt 12 in den jeweiligen Schlitz 11a der Lehre 11 einführen und den Schnitt tätigen. Nach ausgeführtem Schnitt kann dies dem Rechner 16, beispielsweise durch ein betätigen des Fussschalters 18, mitgeteilt werden, worauf der Rechner 16 die Verfahreinrichtung 10 bzw. die Schnittlehre 11 zur nächsten Schneidposition verfährt, so dass der Chirurge den nächsten Schnitt tätigen kann. Dadurch, dass der Rechner 16 bzw. die Schnittlehre 11 sowohl die Lage des Schnittes als auch dessen Ausrichtung genau vorgibt, können die Resektionsschnitte sehr präzise der Geometrie des einzusetzenden Implantates folgend ausgeführt werden. Dieses Verfahren wird vom Chirurgen als grosse Erleichterung empfunden, da er sich beim Schneiden nicht mehr um die Lage des Schnittes zu kümmern braucht und daher seine ganze Aufmerksamkeit auf den Schnitt selbst richten kann, insbesondere auch darauf, dass keine Bänder oder sonstigen Weichteile während dem Schneiden beschädigt werden. Die erfindungsgemässe Vorrichtung ermöglicht es zudem auch einem noch wenig erfahrenen Chirurgen den Femur 1 sowie die Tibia 2 problemlos, genau zu schneiden, und das Implantat einzusetzen.

Die Figuren 13a,13b,13c offenbaren ein Gesamtsystem zum Einsetzen einer Knieprothese, das keiner Schneidlehre 11 mehr bedarf, da die Lage der Sägevorrichtung 14 und damit die Lage des Sägeblattes 12 direkt von der Verfahreinrichtung 10 angesteuert und bestimmt wird.

Am Basisbalken 10g der Verfahreinrichtung 10 ist, wie in Fig. 13c in einer Seitenansicht dargestellt, eine Drehverstelleinrichtung 13q angeordnet, welche als eine Einrastvorrichtung 13a ausgestaltet ist. Diese Einrastvorrichtung 13a weist über deren Umfang verteilt angeordnete Einraststellen 13n auf, wobei jede Einraststelle 13n einen fest vorgegebenen Schwenkwinkel des Arms 13c in Schwenkrichtung 13m festlegt. Eine Rändelschraube 13b erlaubt den Schraubenschaft 13o zu heben bzw. zu senken. Die Drehverstelleinrichtung 13q könnte an Stelle der Einrastvorrichtung 13a auch einen motorischen Antrieb aufweisen, welcher einen vorgebbaren Drehwinkel einzustellen erlaubt. Eine derartige, motorisch angetriebene Drehverstelleinrichtung 13q umfasst vorzugsweise zudem einen Drehwinkelsensor, welcher den Drehwinkel erfasst, so dass der einzunehmende Winkel der Drehverstelleinrichtung 13q beispielsweise mit Hilfe einer Regelvorrichtung oder eines Computers vorgebbar ist.
Wie in Fig. 13b dargestellt, ist der Arm 13c über ein in Bewegungsrichtung 13d bewegliches Gelenk mit einem zweiten Arm 13f verbunden, welcher seinerseits über ein in Bewegungsrichtung 13g bewegliches Gelenk mit dem dritten Arm 13h verbunden ist. Der dritte Arm 13h bildet einerseits eine achsiale Befestigung 13i für die Sägevorrichtung 14 aus und andererseits eine Führung 13k mit Schlitz 131 für das Sägeblatt 12. Der derart ausgestaltete Haltearm 13 erlaubt das Sägeblatt 12 in einer Ebene, vorzugsweise in der Sägeblattebene, beweglich zu führen bzw. zu schwenken. Das Sägeblatt 12 bildet eine Sägeblattebene aus und ist in dieser Ebene verschiebbar gelagert. Der Arm 13 kann derart ausgestaltet sein, dass er eine Federkraft aufweist, so dass beim Zuführen des Sägeblattes 12 zur Kondyle 1a hin eine zunehmende rückstellende Kraft auf die Sägevorrichtung 14 bewirkt wird. Um eine derartige Federkraft zu erzeugen könnten beispielsweise Drehfedern in den Gelenken des Arms 13 angeordnet sein. Im dargestellten Ausführungsbeispiel ist die Führung 13k fest mit dem Arm 13h verbunden. Die Führung 13k könnte jedoch auch über ein Gelenk am Arm 13h angelenkt sein, so dass die Führung 13k zur Befestigung 13i hin schwenkbar gelagert ist. Durch diese Massnahme kann das Sägeblatt 12 tiefer in den zu schneidenden Körper eindringen.

Die Sägevorrichtung 14 weist einen Handgriff 14a auf, welcher, zwecks Verminderung zu starker durch die Bedienung hervorgerufener Momente, bezüglich dem Haltearm 13 in Richtung 14g um die Achse 14h schwenkbar ist. Der Handgriff 14a könnte zudem um eine Drehachse 13p schwenkbar gelagert sein. Der Handgriff 14a dient somit der Bewegungseinleitung in Richtung 14c und in Schwenkrichtung 14d um die Achse 13p. Dadurch ist die Lage des Haltegriffes 14a in vertikaler Schwenkrichtung unabhängig von der Stellung des Sägeblattes 12. Die Neigung des Haltearms 13 bezüglich dem Basisbalken 10g ist, wie in Fig. 13c dargestellt, durch drehen des Arms 13c um die Drehachse 13e der Einrastvorrichtung möglich. Ansonst wird die Lage des Sägeblattes 12 durch die vom Rechner 16 angesteuerte Verfahreinrichtung 10 bestimmt. Da das Sägeblatt 12 in Verlaufsrichtung 14c relativ lang ausgestaltet ist wird die Führung 13k vorzugsweise mit einem Schlitz 131 versehen, um das relativ dünne Sägeblatt 12 in einer definierten Position zu führen, und um ein Durchbiegen des Sägeblattes 12 zu vermeiden. Da der Haltearm 13 zusammen mit der Sägevorrichtung 14 relativ grosse Kräfte auf die Verfahreinrichtung 10 bzw. die Basisplatte 3 auswirken könnte, ist in dem mit Fig. 13a dargestellten Ausführungsbeispiel ein Gestell 15 vorgesehen, welches eine Seilaufwickelvorrichtung 14f umfasst sowie ein Seil 14e, dem die Aufgabe zukommt, eine zumindest die Schwerkraft der Sägevorrichtung 14 kompensierende Gegenkraft F zu erzeugen. Das Gestell 15 umfasst einen Galgen 15a, eine vertikale Stange 15b, ein Untergestellt 15c sowie Räder 15d. Am Gestell 15 ist zudem die Zu- und Ableitung 14b zum Antrieb der Sägevorrichtung 14 angeordnet. Weiter ist der Rechner 16 mit Bildschirm 16a und Tastatur 16b am Gestell 15 befestigt. Zudem ist die Antriebsvorrichtung 17 am Gestell 15 befestigt, wobei die beiden Rändelschrauben 5v, 10f über die biegsame Welle 17a von der Antriebsvorrichtung 17 angetrieben sind.

Der dargestellte Haltearm 13 könnte auch derart ausgestaltet sein, dass dieser Sensoren aufweist, welche die Winkel in Bewegungsrichtung 13d, 13g sowie 13m zu erfassen erlauben, um die genaue Lage des Sägeblattes 12 zu erfassen oder um mit einem an Stelle des Sägeblattes 12 angeordneten Tastkopf die Lage und Geometrie der Kondyle 1a auszumessen.

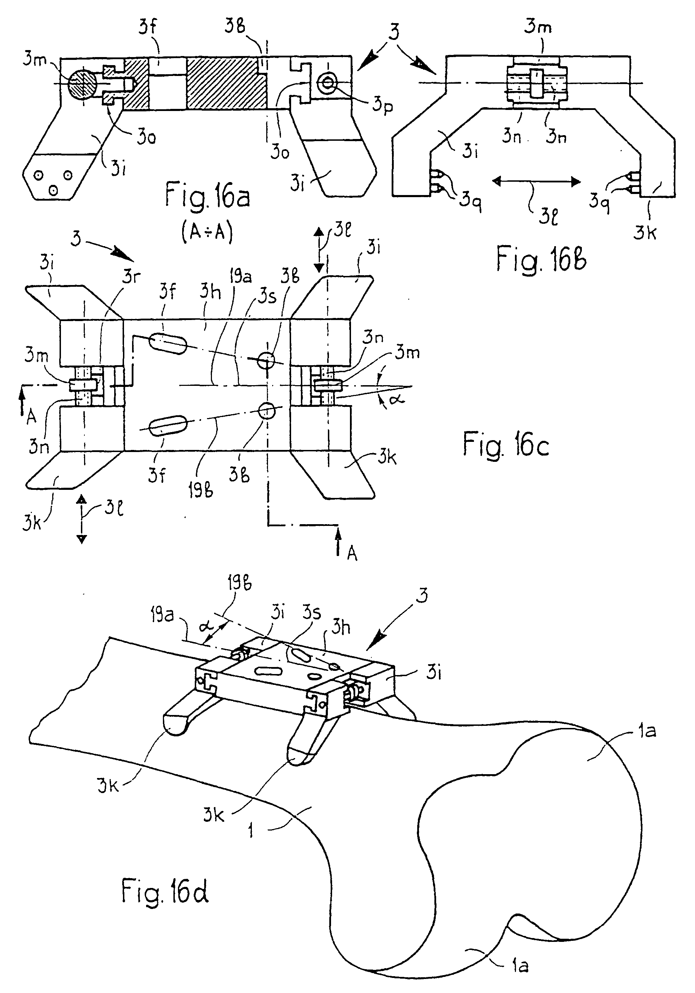

Die Figuren 16a bis 16d offenbaren ein weiteres Ausführungsbeispiel einer am Femur 1 verankerbaren Basisplatte 3 bzw. einer Basisvorrichtung 3. Diese Basisvorrichtung 3 umfasst eine Basisplattform 3h mit Längsachse 3s, an welcher vier in Richtung 31 verschiebbar gelagerte Beine 3i,3k angeordnet sind. Die Beine 3i,3k sind an in Richtung 31 verlaufenden Nuten 3o verschiebbar gelagert. Eine Welle 3n mit Aussengewinde greift in ein Innengewinde der Beine 3i,3k ein. Die Welle 3n weist einen seitlich zugänglichen Schraubenkopf 3p auf. Das Gewinde im Bein 3i ist als Linksgewinde, das Gewinde im Bein 3k als Rechtsgewinde ausgestaltet, wobei das Gewinde der Welle 3n entsprechend zum Eingriff angepasst ausgestaltet ist. Dadurch bewegen sich jeweils zwei nebeneinander angeordnete Beine 3i,3k während dem Drehen am Schraubenkopf 3p entweder aufeinander zu oder voneinander weg. Die Welle 3n weist in der Mitte einen zylinderförmigen, den Durchmesser der Welle 3n übersteigenden Teilabschnitt 3m auf, welcher in einem Zwischenraum 3r der Basisplattform 3h angeordnet ist, und welcher in Verschieberichtung 31 beidseitig mit geringem Spiel an der Basisplattform 3h anliegt und dadurch die Lage der Welle 3n bezüglich der Basisplattform 3h in Richtung 31 festlegt und daher als Zentrierelement 3m dient. Fig. 16b zeigt in einer Seitenansicht zwei gegenüberliegende Beine 3i,3k, welche an den gegenüberliegenden Innenflächen in Verschieberichtung 31 vorstehende Spitzen 3q aufweisen, welche zum Eindringen in den Femur 1 bestimmt sind. Wie in Fig. 16d dargestellt wird die Basisvorrichtung 3 derart am Femur 1 befestigt, dass diese vorerst im Verlaufsrichtung der Femurachse 19a auf den Femur 1 aufgelegt wird und danach die gegenüberliegenden Beine 3i,3k durch Drehen der Welle 3n einander angenähert werden, bis die Spitzen 3q in den Femur 1 eindringen und die Basisvorrichtung 3 sicher mit dem Femur 1 verbunden ist. In einer vorteilhaften Ausgestaltung weist die Welle 3n beidseitig einen Schraubenkopf 3p auf, so dass die Welle 3n wahlweise von einem der beiden Beine 3i,3k her betätigbar ist. Ein Vorteil der dargestellten Ausführungsform einer Basisvorrichtung 3 ist darin zu sehen, dass diese nach dem Befestigen auf dem Femur 1 in Richtung der Femurachse 19a beziehungsweise in Richtung des intramedulären Hohlraumes des Femurs 1 verläuft. Somit weist die Basisvorrichtung 3 eine intrameduläre Verlaufsrichtung auf, ohne jedoch einen intramedulär angeordneten Körper zu verwenden.

Die beiden Ausnehmungen 3b,3f sind ähnlich wie in der Basisplatte 3 gemäss Fig. 1a ausgestaltet und dienen zum Befestigen des Referenzkörpers 5 Mittels eines Bajonettverschlusses. Fig. 16c zeigt in einer Aufsicht die Anordnung der Ausnehmungen 3b,3f auf der Basisplattform 3h. Die beiden unteren Ausnehmungen 3b,3f definieren eine Gerade 19b, welche die durch die Mitte der Basisplattform 3h verlaufende Gerade 19a, 3s unter einem Winkel α schneidet. Dieser Winkel α liegt vorzugsweise im Bereich von 6 ± 2 Grad. Die Gerade 19a entspricht bei auf dem Femur 1 befestigter Basisvorrichtung 3 dem Verlauf der anatomischen Achse 19a des Femurs 1. Statistische Erhebungen haben ergeben, dass die Belastungsachse 19b etwa 6 Grad vom Verlauf der anatomischen Achse 19a abweicht, so dass die in Fig. 16c dargestellte Achse 19b bei auf dem Femur 1 fixierter Basisvorrichtung 3 etwa dem Verlauf der Belastungsachse 19b entspricht. Die Basisvorrichtung 3 weist zwei Paare von Ausnehmungen 3b,3f auf, wobei das oberhalb der Geraden 19a, 3s angeordnete Paar, wie in Fig. 16d dargestellt, beim Femur 1 eines rechten Beines verwendet wird, wogegen das untere Paar beim Femur 1 eines linken Beines zu verwenden ist, um bei vorgegebener anatomischen Achse 19a des Femurs 1 den Verlauf der Belastungsachse 19b ungefähr vorzugeben.

## Patentansprüche

1. Vorrichtung zur Festlegung und Ausführung von Resektionsschnitten am Femur (1) zur Vorbereitung einer Implantation einer Kniegelenktotalendoprothese, mit einer lösbar im distalen Bereich des Femurs (1) arretierbaren Referenzvorrichtung (5), deren Ausrichtung bezüglich dem Femur (1) lagegenau positionierbar ist, und einer mit der Referenzvorrichtung (5) verbundenen, bezüglich dieser verstellbaren Verstelleinrichtung (10), die ein linear verstellbares Basisteil (10g) zum Befestigen eines Instrumentes (11, 14, 101) aufweist, **dadurch gekennzeichnet, dass** die Verstelleinrichtung (10) mittels einer ersten, einen motorischen Antrieb (17d) aufweisenden Antriebsvorrichtung (5v, 17) linear in Richtung (10b) einer ersten Achse (x) eines durch die Referenzvorrichtung festgelegten Koordinatensystems (X, Y, Z) verschiebbar ist und das Basisteil (10g) mittels einer zweiten, einen motorischen Antrieb (17d) aufweisenden Antriebsvorrichtung (10f, 17) in Richtung (10d) einer zweiten Achse (y) des Koordinatensystems (X, Y, Z) linear verstellbar ist.

2. Vorrichtung nach Anspruch 1, **dadurch gekennzeichnet, dass** sowohl die erste wie auch die zweite Antriebsvorrichtung (5v, 17;10f, 17) einen elektromotorischen Antrieb (17d) aufweisen.

3. Vorrichtung nach Anspruch 1 oder 2, **dadurch gekennzeichnet, dass** die motorischen Antriebe (17d) von einem Rechner (16) gesteuert sind, dem vorzugsweise ein Bildschirm (16a) und eine Eingabetastatur (16b) zugeordnet sind.

4. Vorrichtung nach Anspruch 1, **dadurch gekennzeichnet, dass** die Referenzvorrichtung (5) zumindest die erste Achse (x) des Koordinatensystems (X, Y, Z) festlegt, wobei diese erste Achse (x) bei bezüglich des Femurs (1) ausgerichteter Referenzvorrichtung (5) im wesentlichen in Richtung der Belastungsachse (19b) des Femurs (1) verläuft.

5. Vorrichtung nach Anspruch 4, **dadurch gekennzeichnet, dass** die Verstelleinrichtung (10) die zweite Achse (y) des Koordinatensystems (X, Y, Z) festlegt, wobei die erste und zweite Achse (x,y) senkrecht zueinander verlaufen und eine Ebene aufspannen, in welcher im wesentlichen die Belastungsachse (19b) des Femurs (1) liegt.

6. Vorrichtung nach einem der Ansprüche 1-5, **dadurch gekennzeichnet, dass** die Referenzvorrichtung (5) eine in Richtung (10b) der ersten Achse (x) des Koordinatensystems (X., Y, Z) verlaufende Linearführung (5z) aufweist, dass die Verstelleinrichtung (10) eine in die Linearführung (5z) passende, in der genannten Richtung (10b) der ersten Achse (x) des Koordinatensystems (X, Y, Z) verschiebbare Stange umfasst, welche insbesondere als eine Zahnstange (10a) ausgebildet ist, und dass die erste Antriebsvorrichtung (5v, 17) auf die Stange (10a) einwirkt und ein Verfahren der Stange (10a) bezüglich der Referenzvorrichtung (5) erlaubt.

7. Vorrichtung nach einem der Ansprüche 1-6, **dadurch gekennzeichnet, dass** die erste Antriebsvorrichtung (5v, 17) bzw. die zweite Antriebsvorrichtung (10f, 17) entweder direkt an der Referenzvorrichtung (5), bzw. direkt an der Verstelleinrichtung (10) angeordnet ist oder bezüglich der Referenzvorrichtung (5) bzw. der Verstelleinrichtung (10) beabstandet angeordnet und über eine biegsame Welle (17a) wirkungsmässig mit der Referenzvorrichtung (5) bzw. der Verstelleinrichtung (10) verbunden ist.

8. Vorrichtung nach einem der Ansprüche 1-7, **dadurch gekennzeichnet, dass** am Basisteil (10g) der Verstelleinrichtung (10) zumindest ein Messfühler (101) lösbar befestigbar ist, um die Lage der Kondyle (1a) des Femurs (1) abzutasten.

9. Vorrichtung nach einem der Ansprüche 1-7, **dadurch gekennzeichnet, dass** am Basisteil (10g) der Verstelleinrichtung (10) eine Schneidvorrichtung (11, 14), insbesondere eine Schneidlehre (11) zum Führen eines Sägeblattes (12), oder eine Sägevorrichtung (14) mit einem Sägeblatt (12), befestigt ist.

10. Vorrichtung nach Anspruch 9, **dadurch gekennzeichnet, dass** das Sägeblatt (12) der Sägevorrichtung (14) eine Sägeblattebene definiert, dass die Sägevorrichtung (14) mit einem Verbindungsmittel (13) am Basisteil (10g) der Verstelleinrichtung (10) befestigt ist, und dass das Verbindungsmittel (13) sowie die Sägevorrichtung (14) derart ausgestaltet sind, dass das Sägeblatt (12) ausschliesslich in der Sägeblattebene verschiebbar gelagert ist.

11. Vorrichtung nach Anspruch 10, **dadurch gekennzeichnet, dass** das Verbindungsmittel (13) als ein schwenkbarer Arm (13f,13h) oder eine zweiachsige Teleskopführung ausgestaltet ist.

12. Vorrichtung nach Anspruch 10 oder 11, **dadurch gekennzeichnet, dass** ein Handgriff (14a) derart an der Sägevorrichtung (14) angelenkt ist, dass die räumliche Lage des Handgriffes (14a) die Ausrichtung des Sägeblattes (12) bezüglich der Schnittebene nicht beeinflusst.

13. Vorrichtung nach einem der Ansprüche 10-12, **dadurch gekennzeichnet, dass** das Verbindungsmittel (13) über eine, eine Drehachse (13e) aufweisende Drehverstelleinrichtung (13q) mit dem Basisteil (10g) der Verstelleinrichtung (10) verbunden ist, und dass die Drehverstelleinrichtung (13q) ein Verschwenken des Verbindungsmittels (13) bezüglich der Verstelleinrichtung (10) um einen Drehwinkel (13m) erlaubt.

14. Vorrichtung nach Anspruch 13, **dadurch gekennzeichnet, dass** die Drehverstelleinrichtung als eine mechanische Einrastvorrichtung (13q) ausgestaltet ist, die Arretierelemente (13b,13o) aufweist, um den Drehwinkel der Drehachse (13e) in vorbestimmten Stellungen zu arretieren.

15. Vorrichtung nach Anspruch 13, **dadurch gekennzeichnet, dass** die Drehverstelleinrichtung (13q) einen motorischen Antrieb umfasst, welcher einen vorgebbaren Drehwinkel einzustellen erlaubt, und dass die Drehverstelleinrichtung (13q) vorzugsweise einen Drehwinkelsensor umfasst, um den Drehwinkel zu erfassen.

16. Vorrichtung nach einem der Ansprüche 1-15, **dadurch gekennzeichnet, dass** ein Verankerungsteil (3) vorgesehen ist, welches fest am Femur (1) befestigbar ist, und dass das Verankerungsteil (3) zusammen mit der Referenzvorrichtung (5) einen lösbaren Verschluss ausbildet, welcher insbesondere als ein Bajonettverschluss ausgestaltet ist.

17. Vorrichtung nach einem der Ansprüche 1-16, **dadurch gekennzeichnet, dass** die Referenzvorrichtung (5) ein lösbar im distalen Bereich des Femurs (1) arretierbares Basisteil (5a) sowie einen vorzugsweise gelenkig und/oder verschiebbar mit dem Basisteil (5a) verbundenen Referenzkörper (5o) aufweist, dessen Ausrichtung bezüglich dem Femur (1) lagegenau positionierbar ist, und
dass ein zwischen dem Referenzkörper (5o) und dem Basisteil (5a) wirkendes Betätigungsmittel (51, 5r) zum Fixieren deren gegenseitiger Lage vorgesehen ist.

18. Vorrichtung nach Anspruch 17, **dadurch gekennzeichnet, dass** das Basisteil (5a) bezüglich dem Referenzkörper (5o) zumindest um eine bei am Femur (1) arretiertem Basisteil (5a) im wesentlichen in Richtung der Belastungsachse (19b) des Femurs (1) verlaufende Achse (5i) schwenkbar gelagert ist.

19. Vorrichtung nach Anspruch 16, **dadurch gekennzeichnet, dass** das Verankerungsteil (3) eine Dreipunktabstützung (3a) aufweist, welche zur Auflage auf dem Femur (1) bestimmt ist.

20. Vorrichtung nach Anspruch 16 oder 17, **dadurch gekennzeichnet, dass** das Verankerungsteil (3) eine Basisplattform (3h) mit einer Längsachse (3s) umfasst, dass zumindest zwei Beine (3k,3i) bezüglich der Basisplattform (3h) verschieblich gelagert sind und etwa senkrecht zur Längsachse (3s) gegenüberliegend angeordnet sind, und dass die Beine (3k,3i) vorstehende Spitzen (3q) aufweisen, welche zum Femur (1) ausgerichtet verlaufend angeordnet sind und zum Eindringen in den Femurknochen bestimmt sind.

21. Vorrichtung nach einem der Ansprüche 1-20, **dadurch gekennzeichnet, dass** ein Ausrichtstab (7) in der Richtunq einer Achse (x) des Koordinatensystems (X, Y, Z) verlaufend und um eine in der Richtung der dritten Achse (z) des Koordinatensystems (X, Y, Z) verlaufende Drehachse drehbar am Referenzkörper (5o) gelagert ist.

## Claims

1. Device for localizing and executing resection cuts on the femur (1) for preparing an implantation of a total endoprosthetic knee joint, comprising a reference device (5) which can be detachably locked in the distal area of the femur (1) and whose alignment can be accurately positioned relative to the femur (1), and an adjustment device (10) which is connected to the reference device (5), can be adjusted relative to the latter, and has a base part (10g) which can be linearly adjusted for attachment of an instrument (11, 14, 101), **characterized in that** the adjustment device (10) can be displaced, by means of a first drive device (5v, 17) comprising a motor drive (17d), linearly in the direction (106) of a first axis (x) of a system of coordinates (X, Y, Z) determined by the reference device, and the base part (10g) , by means of a second drive device (10f, 17) comprising a motor drive (17d), can be adjusted linearly in the direction (10d) of a second axis (y) of the system of coordinates (X, Y, Z).

2. Device according to Claim 1, **characterized in that** both the first and second drive devices (5v, 21; 10f, 17) comprise an electromotive drive (17d).

3. Device according to Claim 1 or 2, **characterized in that** the motor drives (17d) are controlled by a computer (16), to which a screen (16a) and an input keyboard (16b) are preferably assigned.

4. Device according to Claim 1, **characterized in that** the reference device (5) determines at least the first axis (x) of the system of coordinates (X, Y, Z), in which this first axis (x) extends essentially in the direction of the weight-bearing axis (19b) of the femur (1) when the reference device (5) is aligned relative to the femur (1).

5. Device according to Claim 4, **characterized in that** the adjustment device (10) determines the second axis (y) of the system of coordinates (X, Y, Z), the first and second axes (x, y) extending perpendicular to each other and enclosing a plane in which the weight-bearing axis (19b) of the femur (1) essentially lies.

6. Device according to one of Claims 1 - 5, **characterized in that** the reference device (5) has a linear guide (5z) extending in the direction (10b) of the first axis (x) of the system of coordinates (X, Y, Z), **in that** the adjustment device (10) comprises a rod which fits into the linear guide (5z) and can be displaced in said direction (10b) of the first axis (x) of the system of coordinates (X, Y, Z), which rod is designed in particular as a toothed rod (10a) , and **in that** the first drive device (5v, 17) acts on the rod (10a) and permits a movement of the rod (10a) relative to the reference device (5).

7. Device according to one of Claims 1 - 6, **characterized in that** the first drive device (5v, 17) and the second drive device (10f, 17) are either arranged directly on the reference device (5) and directly on the adjustment device (10), respectively, or are arranged at a distance from the reference device (5) and the adjustment device (10), respectively, and operationally connected to the reference device (5) and the adjustment device (10), respectively, via a flexible shaft (17a).

8. Device according to one of Claims 1 - 7, **characterized in that** at least one measurement sensor (101) can be arranged detachably on the base part (10g) of the adjustment device (10) in order to detect the position of the condyles (1a) of the femur (1).

9. Device according to one of Claims 1 - 7, **characterized in that** a cutting device (11, 14), in particular a sawing jig (11) for guiding a saw blade (12), or a sawing device (14) having a saw blade (12), is secured on the base part (10g) of the adjustment device (10).

10. Device according to Claim 9, **characterized in that** the saw blade (12) of the sawing device (14) defines a saw blade plane, **in that** the sawing device (14) is secured with a connection means (13) on the base part (10g) of the adjustment device (10), and **in that** the connection means (13) and the sawing device (14) are designed in such a way that the saw blade (12) is mounted so as to be displaceable exclusively in the saw blade plane.

11. Device according to Claim 10, **characterized in that** the connection means (13) is designed as a swivel arm (13f, 13h) or a double-axis telescopic guide.

12. Device according to Claim 10 or 11, **characterized in that** a hand grip (14a) is articulated on the sawing device (14) in such a way that the spatial position of the hand grip (14a) does not influence the alignment of the saw blade (12) relative to the cutting plane.

13. Device according to one of Claims 10 - 12, **characterized in that** the connection means (13) is connected to the base part (10g) of the adjustment device (10) via a pivot adjustment device (13q) having a pivot axis (13e), and **in that** the pivot adjustment device (13q) permits swivelling of the connection means (13) relative to the adjustment device (10) about a pivot angle (13m).

14. Device according to Claim 13, **characterized in that** the pivot adjustment device is designed as a mechanical locking device (13q) which has stop elements (13b, 13o) in order to stop the pivot angle of the pivot axis (13e) in predetermined positions.

15. Device according to Claim 13, **characterized in that** the pivot adjustment device (13q) comprises a drive motor making it possible to set a predetermined pivot angle, and **in that** the pivot adjustment device (13q) preferably comprises a pivot angle sensor in order to detect the pivot angle.

16. Device according to one of claims 1 - 15, **characterized in that** an anchoring part (3) is provided which can be attached securely on the femur (1), and **in that** the anchoring part (3) and the reference device (5) together form a releasable catch which is designed in particular as a bayonet catch.

17. Device according to one of claims 1 - 16, **characterized in that** the reference device (5) has a base part (5a) which can be detachably stopped in the distal area of the femur (1), and a reference body (50) which is connected to the base part (5a) in preferably an articulated and/or displaceable manner,. and whose alignment can be accurately positioned relative to the femur (1), and **in that** an actuating means (51, 5r) acting between the reference body (5o) and the base part (5a) is provided for fixing the mutual position thereof.

18. Device according to Claim 17, **characterized in that** the base part (5a) is mounted so as to pivot relative to the reference body (5o) at least about an axis (5i) which extends essentially in the direction of the weight-bearing axis (19b) of the femur (1) when the base part (5a) is stopped on the femur (1).

19. Device according to Claim 16, **characterized in that** the anchoring part (3) has a three-point support (3a) which is intended to bear on the femur (1).

20. Device according to Claim 16 or 17, **characterized in that** the anchoring part (3) comprises a base platform (3h) with a longitudinal axis (3s), **in that** at least two legs (3k, 3i) are mounted displaceably relative to the base platform (3h) and are arranged opposite each other approximately perpendicular to the longitudinal axis (3s), and **in that** the legs (3k, 3i) have projecting points (3q) which are arranged extending towards the femur (1) and are intended to penetrate into the femoral bone.

21. Device according to one of claims 1 - 20, **characterized in that** an alignment rod (7) is mounted extending in the direction of an axis (x) of the system of coordinates (X, Y, Z) and can pivot about a pivot axis extending in the direction of the third axis (z) of the system of coordinates (X, Y, Z) on the reference body (5o).

## Revendications

1. Dispositif pour la détermination et la réalisation de coupes de résection sur le fémur (1) pour préparer une implantation d'une endoprothèse totale de l'articulation du genou, comprenant :
- un dispositif de référence (5) pouvant être bloqué dans la région distale du fémur (1) avec possibilité de détachement et dont l'orientation par rapport au fémur (1) peut être positionnée exactement ; et
- un dispositif de réglage (10), relié au dispositif de référence (5) et réglable par rapport à celui-ci, qui présente une partie de base (10g) réglable linéairement pour fixer un instrument (11, 14, 101),
**caractérisé en ce que** :
- le dispositif de réglage (10) est déplaçable linéairement en direction (10b) d'un premier axe (x) d'un système de coordonnées (X, Y, Z) défini par le dispositif de référence, au moyen d'un premier dispositif d'entraînement (5v, 17) présentant un entraînement motorisé (17d) ; et
- la partie de base (10g) est réglable linéairement en direction (10d) d'un second axe (y) du système de coordonnées (X, Y, Z), au moyen d'un second dispositif d'entraînement (10f, 17) présentant un entraînement motorisé (17d).

2. Dispositif selon la revendication 1, **caractérisé en ce qu'**aussi bien le premier que le second dispositif d'entraînement (5v, 17 ; 10f, 17) présentent un entraînement par moteur électrique.

3. Dispositif selon la revendication 1 ou 2, **caractérisé en ce que** les entraînements motorisés (17d) sont commandés par un calculateur (16) auquel sont associés de préférence un écran (16a) et un clavier de saisie (16b).

4. Dispositif selon la revendication 1, **caractérisé en ce que** le dispositif de référence (5) définit au moins le premier axe (x) du système de coordonnées (X, Y, Z), ce premier axe (x) s'étendant essentiellement en direction de l'axe de sollicitation (19b) du fémur (1) lorsque le dispositif de référence (5) est orienté par rapport au fémur (1).

5. Dispositif selon la revendication 4, **caractérisé en ce que** le dispositif de réglage (10) définit le second axe (y) du système de coordonnées (X, Y, Z), le premier et le second axe (x, y) s'étendant perpendiculairement l'un à l'autre et définissant un plan dans lequel se trouve essentiellement l'axe de sollicitation (19b) du fémur (1).

6. Dispositif selon l'une des revendications 1 à 5, **caractérisé en ce que** :
- le dispositif de référence (5) présente un guidage linéaire (5z) s'étendant en direction (10b) du premier axe (x) du système de coordonnées (X, Y, Z) ;
- le dispositif de réglage (10) comporte une tige adaptée au guidage linéaire (5z) et mobile dans la direction mentionnée (10b) du premier axe (x) du système de coordonnées (X, Y, Z), ladite tige étant réalisée en particulier comme tige à crémaillère (10a) ; et
- le premier dispositif d'entraînement (5v, 17) agit sur la tige (10a) et permet un déplacement de la tige (10a) par rapport au dispositif de référence (5).

7. Dispositif selon l'une des revendications 1 à 6, **caractérisé en ce que** le premier dispositif d'entraînement (5v, 17) ou le second dispositif d'entraînement (10f, 17) est agencé soit directement sur le dispositif de référence (5), soit directement sur le dispositif de réglage (10), ou est agencé à une certaine distance par rapport au dispositif de référence (5) ou au dispositif de réglage (10), et est relié fonctionnellement au dispositif de référence (5) ou au dispositif de réglage (10) via un arbre flexible (17a).

8. Dispositif selon l'une des revendications 1 à 7, **caractérisé en ce qu'**au moins un capteur de mesure (101) peut être fixé de manière détachable sur la partie de base (10g) du dispositif de réglage (10) pour explorer la position des condyles (1a) du fémur (1).

9. Dispositif selon l'une des revendications 1 à 7, **caractérisé en ce qu'**un dispositif de coupe (11, 14), en particulier un gabarit de coupe (11), est fixé sur la partie de base (10g) du dispositif de réglage (10) pour guider une lame de scie (12) ou un dispositif de sciage (14) comportant une lame de scie (12).

10. Dispositif selon la revendication 9, **caractérisé en ce que** :
- la lame de scie (12) du dispositif de sciage (14) définit un plan de lame de scie ;
- le dispositif de sciage (14) est fixé sur la partie de base (10g) du dispositif de réglage (10) avec un moyen de liaison (13) ; et
- le moyen de liaison (13) ainsi que le dispositif de sciage (14) sont réalisés de sorte que la lame de scie (12) est montée mobile en translation exclusivement dans le plan de la lame de scie.

11. Dispositif selon la revendication 10, **caractérisé en ce que** le moyen de liaison (13) est réalisé comme un bras pivotant (13f, 13h) ou comme un guidage télescopique à deux axes.

12. Dispositif selon la revendication 10 ou 11, **caractérisé en ce qu'**une poignée (14a) est montée avec articulation sur le dispositif de sciage (14) de sorte que la position de la poignée (14a) dans l'espace n'influe pas sur l'orientation de la lame de scie (12) par rapport au plan de coupe.

13. Dispositif selon l'une des revendications 10 à 12, **caractérisé en ce que** le moyen de liaison (13) est relié à la partie de base (10g) du dispositif de réglage (10) via un dispositif de réglage rotatif (13q) présentant un axe de rotation (13e) et **en ce que** le dispositif de réglage rotatif (13q) permet un pivotement d'un angle de rotation (13m) du moyen de liaison (13) par rapport au dispositif de réglage (10).

14. Dispositif selon la revendication 13, **caractérisé en ce que** le dispositif de réglage rotatif est réalisé comme un dispositif à enclenchement mécanique (13q) qui présente des éléments de blocage (13b , 13o) pour bloquer l'angle de rotation de l'axe de rotation (13e) dans des positions prédéterminées.

15. Dispositif selon la revendication 13, **caractérisé en ce que** le dispositif de réglage rotatif (13q) comporte un entraînement motorisé qui permet d'ajuster un angle de rotation pouvant être prescrit, et **en ce que** le dispositif de réglage rotatif (13q) comporte de préférence un capteur d'angle de rotation pour saisir l'angle de rotation.

16. Dispositif selon l'une des revendications 1 à 15, **caractérisé en ce qu'**il est prévu une pièce d'ancrage (3) qui peut être fixée fermement au fémur (1), et **en ce que** la pièce d'ancrage (3) forme, conjointement avec le dispositif de référence (5), une fermeture détachable qui est réalisée en particulier comme fermeture à baïonnette.

17. Dispositif selon l'une des revendications 1 à 16, **caractérisé en ce que** :
- le dispositif de référence (5) présente une partie de base (5a) pouvant être bloquée de façon détachable dans la région distale du fémur (1) ainsi qu'un corps de référence (50) relié à la partie de base (5a) de préférence avec articulation et/ou mobile en translation et dont l'orientation par rapport au fémur (1) peut être positionnée exactement ; et
- il est prévu un moyen d'actionnement (5l, 5r) agissant entre le corps de référence (50) et la partie de base (5a) pour fixer leur position réciproque.

18. Dispositif selon la revendication 17, **caractérisé en ce que**, par rapport au corps de référence (50), la partie de base (5a) est montée avec faculté de pivotement au moins autour d'un axe (5i) s'étendant essentiellement en direction de l'axe de sollicitation (19b) du fémur (1) lorsque la partie de base (5a) est bloquée sur le fémur (1).

19. Dispositif selon la revendication 16, **caractérisé en ce que** la pièce d'ancrage (3) présente un soutien à trois points (3a) qui est destiné à l'appui sur le fémur (1).

20. Dispositif selon la revendication 16 ou 17, **caractérisé en ce que** :
- la pièce d'ancrage (3) comporte une plate-forme de base (3h) comprenant un axe longitudinal (3 s) ;
- au moins deux plaquettes (3k, 3i) sont montées mobiles en translation par rapport à la plate-forme de base (3h) et sont agencées à l'opposé approximativement perpendiculairement à l'axe longitudinal (3s) ; et
- les plaquettes (3k, 3i) présentent des pointes (3q) qui dépassent, qui sont agencées en s'étendant avec orientation vers le fémur (1) et qui sont destinées à pénétrer dans l'os du fémur.

21. Dispositif selon l'une des revendications 1 à 20, **caractérisé en ce qu'**une barre d'orientation (7) est montée sur le corps de référence (50), en s'étendant en direction d'un axe (x) du système de coordonnées (X, Y, Z) et avec faculté de rotation autour d'un axe de rotation s'étendant en direction d'un troisième axe (z) du système de coordonnées (X, Y, Z).
